(19) **Europäisches Patentamt European Patent Office Office européen des brevets**

(11) **EP 4 648 054 A1**

(12) **EUROPEAN PATENT APPLICATION**
published in accordance with Art. 153(4) EPC

(43) Date of publication:
**12.11.2025 Bulletin 2025/46**

(21) Application number: **23915028.7**

(22) Date of filing: **28.12.2023**

(51) International Patent Classification (IPC):
*G16B 40/10* (2019.01)     *G16B 45/00* (2019.01)
*G16B 20/00* (2019.01)     *G06N 3/04* (2023.01)
*G16B 40/20* (2019.01)     *G16B 25/20* (2019.01)

(52) Cooperative Patent Classification (CPC):
G06N 3/04; G16B 20/00; G16B 25/20; G16B 40/10;
G16B 40/20; G16B 45/00

(86) International application number:
**PCT/KR2023/021950**

(87) International publication number:
**WO 2024/147568 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2023  KR 20230001518**
           **11.01.2023  KR 20230003846**

(71) Applicant: **Seegene, Inc.**
    **Seoul 05548 (KR)**

(72) Inventors:
• **SON, Hyungsuk**
  **Seoul 05548 (KR)**
• **PARK, Bong Gyun**
  **Seoul 05548 (KR)**

(74) Representative: **Viering, Jentschura & Partner mbB**
    **Patent- und Rechtsanwälte**
    **Am Brauhaus 8**
    **01099 Dresden (DE)**

(54) **METHOD FOR OBTAINING MOLECULAR DIAGNOSTIC ANALYSIS RESULTS, METHOD FOR OBTAINING MODEL TO ESTIMATE MOLECULAR DIAGNOSTIC ANALYSIS RESULTS, AND COMPUTER DEVICE FOR PERFORMING SAME**

(57)     Provided according to one embodiment is a method for acquiring molecular diagnostic analysis results, performed by a computer device using a memory, a processor, and one or more programs stored in the memory and configured to be executed by the processor, the method comprising the steps of: acquiring a dataset representing results of the amplification reaction for a target analyte in the sample; calculating the shape similarity for each reference pattern by comparing the target curve in the dataset to multiple pre-established reference patterns; and providing the shape similarity for each reference pattern to a pre-trained estimation model, and acquiring, from the estimation model, molecular diagnostic analysis results including at least one of the Ct of the target curve, the quantitative value of the target analyte in the sample, the positive/negative reading result for the target analyte in the sample, and the suitability assessment result of oligonucleotide candidates used in the amplification reaction.

*FIG.1*

**Description**

[Technical Field]

The disclosure relates to a method for obtaining molecular diagnostic analysis results, a method for obtaining a model to estimate molecular diagnostic analysis results, and a computer device for performing the same.

[Background Art]

Currently, molecular diagnosis is a rapidly growing field in the in vitro diagnostic market for early diagnosis of diseases. Among the molecular diagnostic methods, methods using nucleic acids are usefully used for diagnosing causal genetic factors caused by infections by viruses, bacteria, etc., based on their high specificity and sensitivity.

Most diagnostic methods using nucleic acids use nucleic acid amplification reactions that amplify target nucleic acids (e.g., viral or bacterial nucleic acids). As a representative example, a polymerase chain reaction (PCR) among the nucleic acid amplification reactions performs a repeated cycle process of denaturation of doublestranded DNA, annealing of an oligonucleotide primer to a DNA template, and primer extension by DNA polymerase (Mullis et al., U.S. Patent Nos. 4,683,195, 4,683,202, and 4,800,159; Saiki et al., Science 230:1350-1354 (1985)).

As other methods for amplifying nucleic acid, various methods, such as ligase chain reaction (LCR), strand displacement amplification (SDA), nucleic acid sequence-based amplification (NASBA), transcription mediated amplification (TMA), recombinase polymerase amplification (RPA), loop-mediated isothermal amplification (LAMP), and rolling-circle amplification (RCA), have been proposed.

Among the PCR-based technologies, real-time PCR is a technology for detecting target nucleic acids in a sample in real time. In order to detect a specific target nucleic acid, a signal generating means that emits a detectable fluorescent signal in proportion to the amount of target nucleic acids during a PCR reaction is used. The fluorescent signal proportional to the amount of target nucleic acids is detected at each measurement point (cycle) through the real-time PCR, so a dataset including each measurement point and signal values at the measurement points is obtained. An amplification curve or an amplification profile curve indicating the intensity of the detected fluorescent signal with respect to the measurement point is obtained from the dataset.

In general, the amplification curve by the real-time PCR is divided into a baseline region, an exponential region, and a plateau region. The exponential region is a region where the fluorescent signal emitted in proportion to the increase in the PCR amplification product increases, and the plateau region is a region where the increase in the PCR amplification product and the emission of the fluorescent signal reach a saturation state and thus the fluorescent signal no longer increases. The baseline region refers to a region where the fluorescent signal remains constant without change at the early stage of the reaction. Since the baseline region is not sufficient to detect the fluorescent signal emitted by the PCR reaction product, most of the baseline area is occupied by the background signal composed of the fluorescent signal from the reaction sample itself and the fluorescent signal from the measurement system itself, rather than the fluorescent signal by the amplification of the target analyte.

When any environmental causes act during the amplification process, the amplification curve may not be divided into the three regions described above, or even if the amplification curve is divided, there may be differences in the change pattern of the fluorescent signal, the signal values, the measurement point, etc., within each region. These environmental causes include noise, interference, etc., caused by internal or external factors of the reaction sample or the measurement system. When the amplification curve is affected by such environmental causes, it becomes difficult to derive the diagnostic results, which may decrease the diagnostic accuracy.

Various methods have been developed to analyze the diagnostic results based on the data obtained from the amplification reaction. For example, various methods have been developed to analyze the presence or absence of the amplification of the target nucleic acid from the real-time PCR dataset, a cycle threshold (Ct) value that may be used as a basis for assessing the presence or absence of the amplification of the target nucleic acid, and a quantitative value of how much target nucleic acid is present, etc.

Among these, the Ct is mainly used in the process of deriving the diagnostic results. For example, the Ct may be used in the process of determining the presence or absence of the target nucleic acids in the sample from the real-time PCR dataset, in the process of quantitating nucleic acids, such as analyzing an initial concentration of the target nucleic acids, etc. In most cases, the Ct refers to a specific cycle number that appears in the amplification result. For example, the Ct may refer to the cycle number when the signal value measured by the real-time PCR satisfies a predetermined condition.

As a method for obtaining the Ct, there are known a threshold method that arbitrarily set a line parallel to an x-axis (axis of the cycle number) in an amplification curve and determine Ct according to an x-axis value that intersects the amplification curve, a first or second differentiation method that determine Ct according to a maximum value of a first differentiation curve or a second differentiation curve of an amplification curve, etc.

However, the existing methods have the problem in that the difference in the Ct values increases depending on the aspects of the background signal, the noise, or the interference, etc., when the amplification curve includes the above-described background signal, noise, or interference, etc. In addition, the existing methods are performed on an amplification curve that includes a signal dependent on the presence of one type of target nucleic acid. However, there is a limitation in that the existing methods may not be applied when signals dependent on the presence of two or more types of target nucleic acids are mixed.

In addition, the quantitation of the target nucleic acid may relative or absolute. As the absolute quantitation method, there are known a standard curve technique that obtains a standard curve using the amplification curve of the target nucleic acid of which the amount is known, and absolutely quantitates the amount of the unknown sample by comparing the amplification results of the unknown sample with the standard curve, a digital PCR technique that measures precision by the number of replicates of a target to perform absolute quantitation, etc. As the relative quantitation method, there are known a standard curve technique that measures the amount of target from a standard curve for all experimental samples and divides the measured amount of target by the target amount of calibrator such as an untreated control group to perform relative quantitation, a comparative CT technique that compares a Ct value of one target nucleic acid with another target nucleic acid to perform relative quantitation, etc.

In most of the cases described above, the standard curve for the quantitation is required. That is, prior to the quantitation analysis of the target, experiments for obtaining the standard curve should be involved, and works is required to analyze the quantitation using the standard curve. In particular, when multiple different target nucleic acids need to be quantitated using the real-time PCR for the absolute quantitation, the existing methods for the absolute quantitation has the disadvantage of being inefficient in terms of time and cost.

In addition, as a positive/negative reading method for whether the amplification of the target nucleic acid is present, a technique for calculating a basis value such as the above-described Ct or quantitative value from the amplification curve, and determining the presence or absence of the target nucleic acid in the sample based on the calculated basis value, or the like, has been known. As in the process of calculating the Ct or the quantitative value described above, the existing methods have a problem in that the accuracy of target detection deteriorates as the difference in the basis value increases depending on aspects such as the background signal, the noise, or the interference.

Accordingly, there is a need to better estimate molecular diagnostic analysis results, such as Ct, quantitation results of target nucleic acids, or positive/negative reading, by utilizing data obtained by an amplification reaction.

[Disclosure]

[Technical Problem]

According to one embodiment, a problem to be solved includes efficiently or effectively estimating molecular diagnostic analysis results based on data obtained by an amplification reaction to solve the above-described problems and/or limitations.

According to another embodiment, a problem to be solved includes better estimating a Ct of a target analyte based on data obtained by an amplification reaction.

According to still another embodiment, the problem to be solved includes efficiently estimating a quantitative value of a target analyte based on data obtained by an amplification reaction.

According to further embodiment, a problem to be solved includes more accurately estimating a positive/negative determination result for the target analyte in the sample based on data obtained by an amplification reaction.

According to further embodiment, a problem to be solved includes estimating a suitability assessment result of oligonucleotide candidates to be used in an amplification reaction based on data obtained by the corresponding amplification reaction.

However, the problem to be solved by the present disclosure is not limited to that mentioned above, and other problems to be solved that are not mentioned may be clearly understood by those of ordinary skill in the art to which the present disclosure belongs from the following description.

[Technical Solution]

A method for obtaining molecular diagnostic analysis results according to an embodiment of the present disclosure, performed by a computer device using a memory, a processor, and one or more programs stored in the memory and configured to be executed by the processor, the method comprising obtaining a dataset representing results of an amplification reaction for a target analyte in a sample; calculating a shape similarity for each reference pattern by comparing a target curve generated based on the dataset to each of pre-determined multiple reference patterns; and providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

According to one embodiment, wherein the amplification reaction may be based on real-time amplification.

According to one embodiment, wherein the dataset may include a signal value in each of multiple cycles obtained as a result of the amplification reaction or an nth (n is a natural number) derivative result of a curve connecting the signal value in each of the multiple cycles.

According to one embodiment, wherein the multiple reference patterns may be determined based on at least any one selected from a group including an amplification reference pattern in a case where the target analyte is absent in the sample, an amplification reference pattern in cases where one type of target analyte detectable in a single channel is present at a relatively high concentration or a relatively low concentration in the sample, respectively, an amplification reference pattern in cases where two or more types of target analytes detectable in the single channel are present at same concentration or at different concentrations in the sample, respectively, an aspect of a background signal included in a result of the amplification reaction, an aspect of an abnormal signal included in the result of the amplification reaction, and an aspect of a non-specific signal due to an amplification reaction other than an intended amplification.

According to one embodiment, wherein a reference pattern according to the aspect of the abnormal signal may include a reference pattern in at least one of a case where a magnitude of amplitude included in the result of the amplification reaction increases discretely, a case where signal interference is received from another channel, or a case where the magnitude of the amplitude increases linearly.

According to one embodiment, wherein the shape similarity for each reference pattern is calculated by computing a cross correlation between the target curve and each of the multiple reference patterns.

According to one embodiment, wherein a computation of the cross correlation may be performed by at least any one selected from a group including a pre-stored cross correlation scheme, a zero-normalized cross correlation scheme, a normalized cross correlation scheme, and a correlation coefficient scheme.

According to one embodiment, wherein the shape similarity for each reference pattern may be generated as an image type, and the pre-trained estimation model may receive the shape similarity for each reference pattern generated as the image type as an input.

According to one embodiment, wherein, in the shape similarity for each reference pattern, a similarity of the target curve with respect to each reference pattern may be distinguished by color on the image.

According to one embodiment, wherein the shape similarity for each reference pattern of the image type is obtained by measuring the shape similarity at each shift amount for each of the multiple reference patterns, when shifting any one of the target curve and the reference pattern by changing the shift amounts.

According to one embodiment, wherein the pre-trained estimation model includes at least any one selected from a group including a convolutional neural network (CNN), a deep neural network (DNN), a recurrent neural network (RNN), a vision transformer (ViT), and a generative adversarial network (GAN).

According to one embodiment, wherein a range of the Ct, the quantitative value, the positive/negative determination result for the target analyte in the sample, or the suitability assessment result of the oligonucleotide candidates may be partitioned into multiple sections, and each of the multiple sections may be mapped to any one of multiple classes, and when receiving the shape similarity for each reference pattern, the pre-trained estimation model may output a probability value for each of the multiple classes.

According to one embodiment, wherein the pre-trained estimation model may be trained using multiple training datasets, and each training dataset may include (a) training input data including the shape similarity for each reference pattern by comparing the target curve generated based on the dataset representing the result of the amplification reaction for the target analyte in the sample to each of the multiple reference patterns, and (b) training ground truth data including label data for the molecular diagnostic analysis results including at least any one selected from a group including the Ct, the quantitative value, the positive/negative determination result for the target analyte in the sample, and the suitability assessment result of the oligonucleotide candidates.

A computer program stored in a non-transitory computer-readable storage medium according to an embodiment of the present disclosure, wherein the computer program, when executed by at least one processor, includes instructions for causing the at least one processor to perform a method for obtaining molecular diagnostic analysis results, the method comprising: obtaining a dataset representing results of an amplification reaction for a target analyte in a sample; calculating a shape similarity for each reference pattern by comparing a target curve generated based on the dataset to each of pre-determined multiple reference patterns; and providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

A non-transitory computer-readable storage medium storing a computer program according to an embodiment of the present disclosure, wherein the computer program, when executed by at least one processor, includes instructions for causing the at least one processor to perform a method for obtaining molecular diagnostic analysis results, the method comprising: obtaining a dataset representing results of an amplification reaction for a target analyte in a sample; calculating a shape similarity for each reference pattern by comparing a target curve generated based on the dataset to each of pre-determined multiple reference patterns; and providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

A computer device according to an embodiment of the present disclosure, comprising: a memory storing at least one instruction; and a processor; wherein the at least one instruction, when executed by the processor, causes the processor to: obtain a dataset representing results of an amplification reaction for a target analyte in a sample; calculate a shape similarity for each reference pattern by comparing a target curve generated based on the dataset to each of pre-determined multiple reference patterns; and provide the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

A method for obtaining a model to estimate molecular diagnostic analysis results according to an embodiment of the present disclosure, performed by a computer device using a memory, a processor, and one or more programs stored in the memory and configured to be executed by the processor, the method comprising: obtaining multiple training datasets; each training dataset including (a) training input data including a shape similarity for each reference pattern by comparing a target curve generated based on a dataset representing a result of an amplification reaction for a target analyte in a sample to each of pre-determined multiple reference patterns, and (b) training ground truth data including label data for at least any one selected from a group including Ct, a quantitative value, a positive/negative determination result for the target analyte in the sample, and a suitability assessment result of oligonucleotide candidates; and obtaining an estimation model trained to estimate at least any one selected from a group including the Ct, the quantitative value, the positive/negative determination result for the target analyte in the sample, and the suitability assessment result of oligonucleotide candidates, when the shape similarity for each reference pattern is provided using the multiple training datasets

A method for obtaining molecular diagnostic analysis results according to an embodiment of the present disclosure, performed by a computer device using a memory, a processor, and one or more programs stored in the memory and configured to be executed by the processor, the method comprising: obtaining a dataset representing results of an amplification reaction for a target analyte in a sample; calculating a shape similarity for each reference pattern by comparing a target curve generated based on the dataset to each of pre-determined multiple reference patterns; and obtaining molecular diagnostic analysis results for the target analyte in the sample using the shape similarity for each reference pattern.

According to one embodiment, whrein the molecular diagnostic analysis results may include at least any one selected from a group including Ct of the target curve, a quantitative value of the target analyte in the sample, a positive/negative determination result for the target analyte in the sample, and a suitability assessment result of oligonucleotide candidates to be used in the amplification reaction.

A method for calculating a shape similarity for molecular diagnostic analysis according to an embodiment of the present disclosure, performed by a computer device using a memory, a processor, and one or more programs stored in the memory and configured to be executed by the processor, the method comprising: obtaining a dataset representing a result of an amplification reaction; and calculating a shape similarity for each reference pattern by comparing a target curve generated based on the dataset to each of pre-determined multiple reference patterns.

According to one embodiment, wherein the multiple reference patterns may be determined based on at least any one selected from a group including an amplification reference pattern in a case where the target analyte is absent in the sample, an amplification reference pattern in cases where one type of target analyte detectable in a single channel is present at a relatively high concentration or a relatively low concentration in the sample, respectively, an amplification reference pattern in cases where two or more types of target analytes detectable in the single channel are present at same concentration or at different concentrations in the sample, respectively, an aspect of a background signal included in a result of the amplification reaction, an aspect of an abnormal signal included in the result of the amplification reaction, and an aspect of a non-specific signal due to an amplification reaction other than an intended amplification.

According to one embodiment, wherein the shape similarity for each reference pattern may be calculated by computing a cross correlation between the target curve and each of the multiple reference patterns.

According to one embodiment, wherein the shape similarity for each reference pattern may be generated as an image type, and the pre-trained estimation model may receive the shape similarity for each reference pattern generated as the image type as an input.

[Advantageous Effects]

According to one embodiment of the present disclosure, molecular diagnostic analysis results may be estimated more efficiently or effectively from data obtained by an amplification reaction. According to one embodiment, even if an amplification curve includes a background signal, noise, or interference, etc. Ct may be well estimated, which is effective in terms of estimation performance. According to another embodiment, various processes conventionally involved for quantitation analysis, such as experiments for a standard curve, can be omitted at least partially, which is efficient in terms of time and cost. According to another embodiment, even if an amplification curve does not show an ideal positive/negative signal shape, a more accurate positive/negative determination result for the target analyte in the sample may be provided, and various processes conventionally involved for positive/negative reading, such as Ct calculation or background signal correction, can be omitted. According to another embodiment, a suitability assessment result of oligonucleotide candidates may be provided from data obtained by an amplification reaction using oligonucleotide candidates. In this way, various processes conventionally involved for selecting oligonucleotide candidates for detection of a target analyte may be at least partially omitted, which is efficient in terms of time and cost.

In addition, by using an artificial neural network, features included in a dataset that are difficult to analyze by humans may be extracted and used to estimate molecular diagnostic analysis results including at least one of a Ct, a quantitative value, a positive/negative determination result for the target analyte in the sample, or a suitability assessment result of oligonucleotide candidates, which is effective in terms of estimation performance. In addition, data input to an estimation model may be advanced through a preprocessing process that includes shape similarity information between an amplification curve and various reference patterns, instead of applying an amplification curve of a target analyte to a neural network. This makes it easier to extract more meaningful features for estimating the molecular diagnostic analysis results in the estimation model.

According to another embodiment of the present disclosure, the above-described effect is also possible in obtaining molecular diagnostic analysis results other than the above-described Ct, quantitative value, positive/negative determination result for the target analyte in the sample, and suitability assessment result of oligonucleotide candidates.

The effects of the present disclosure are not limited to the effects described above, but should be understood to include all effects that can be inferred from the detailed description or the configuration of the invention recited in the claims.

[Brief Description of Drawings]

FIG. 1 schematically illustrates a block diagram of a computer device according to an embodiment.

FIG. 2 illustrates a modular representation of software implemented by the computer device shown in FIG. 1.

FIG. 3 exemplarily illustrates a conceptual diagram of the process of preprocessing the dataset for training as the input data to be provided to the estimation model according to one embodiment.

FIGS. 4 and 5 each exemplarily illustrate the multiple datasets and the multiple reference patterns according to an embodiment.

FIG. 6 exemplarily illustrates a process of measuring the shape similarity through the computation of the cross correlation according to one embodiment.

FIGS. 7 to 9 each exemplarily illustrate he shape similarity for each reference pattern according to an embodiment, the shape similarity for each reference pattern generated as an image type, and the shape similarity for each reference pattern of an image type according to one embodiment, respectively.

FIG. 10 exemplarily illustrates a conceptual diagram of a process of obtaining multiple training datasets according to one embodiment.

FIG. 11 exemplarily illustrates the multiple training datasets according to one embodiment.

FIG. 12 exemplarily illustrates a conceptual diagram of the process of training the estimation model according to one embodiment

FIG. 13 exemplarily illustrates a schematic structure and operation of a CNN-based deep learning model used for training according to one embodiment.

FIG. 14 illustrates an exemplary flowchart for a computer device according to one embodiment to train the estimation model.

FIG. 15 exemplarily illustrates a conceptual diagram for the process of preprocessing the dataset for estimating the molecular diagnostic analysis results according to one embodiment as the input data to be provided to the estimation model.

FIG. 16 exemplarily illustrates the dataset, the multiple reference patterns, and the shape similarity for each reference pattern for estimating the molecular diagnostic analysis results according to one embodiment.

FIG. 17 exemplarily illustrates a conceptual diagram of the estimation process using the estimation model according to one embodiment.

FIG. 18 exemplarily illustrates a process of providing an estimation result for the molecular diagnostic analysis results according to one embodiment.

FIG. 19 illustrates an exemplary flowchart for estimating the molecular diagnostic analysis results by the computer device according to one embodiment.

FIG. 20 illustrates an exemplary flowchart for obtaining the molecular diagnostic analysis results by the computer device according to one embodiment.

[Mode for Disclosure]

The advantages and features of the embodiments and the methods of accomplishing the embodiments will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims.

In describing embodiments of the present invention, if it is considered that a detailed description of a known function or configuration may unnecessarily obscure the gist of the present invention, the detailed description will be omitted. In addition, the terms described below are terms defined in consideration of functions in the embodiments of the present invention, the terms may vary according to the intention or precedent of a technician working in the field, the emergence of new technologies, and the like. Therefore, the terms used in the present disclosure should be defined based on the meaning of the terms and the overall contents of the present disclosure, not just the name of the terms.

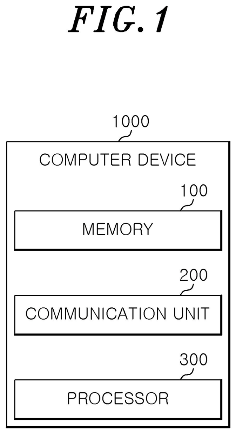

Before describing FIG. 1, the terms used in the present invention will be described.

The term "target analyte" may refer to various substances (e.g., biological substances and non-biological substances). The target analyte may specifically include the biological substances, and more include specifically at least one of nucleic acid molecules (e.g., DNA and RNA), proteins, peptides, carbohydrates, lipids, amino acids, biological compounds, hormones, antibodies, antigens, metabolites, and cells.

The term "sample" refers to biological samples (e.g., cells, tissues, and body fluids) and non-biological samples (e.g., food, water, and soil). Among the samples, the biological samples may include at least one of, for example, viruses, bacteria, tissues, cells, blood (including whole blood, plasma, and serum), lymph, bone marrow fluid, saliva, sputum, swab, aspiration, milk, urine, stool, eye fluid, semen, brain extracts, spinal fluid, joint fluid, thymic fluid, bronchoalveolar lavage fluid, ascites, and amniotic fluid. These samples may or may not include the target analyte described above.

Meanwhile, when the target analyte described above is nucleic acid molecules or include the nucleic acid molecules, a nucleic acid extraction process known in the art may be performed on a sample estimated as including the target analyte (reference: Sambrook, J. et al., Molecular Cloning. A Laboratory Manual, 3rd ed. Cold Spring Harbor Press (2001)).

Meanwhile, as described above, the target analyte may be amplified by various methods. An amplification reaction according to the present disclosure may be based on PCR, LCR, SDA, TMA, NASBA, RCA, Q-Beta Replicase, LAMP, or RPA. The amplification reaction according to one embodiment may be based on real-time amplification, for example, the real-time PCR described above.

According to one embodiment, the amplification reaction for amplifying a signal indicating the presence of the target analyte may be performed by a method (e.g., the real-time PCR method) in which a signal is also amplified while the target analyte is amplified. Alternatively, according to one embodiment, the amplification reaction may be performed by a method (e.g., CPT method) in which only the signal indicating the presence of the target analyte is amplified without the target analyte being amplified. In this way, the amplification reaction may be accompanied by a signal change, and therefore, the degree of progress of this amplification reaction may be evaluated by measuring the signal change. As such a signal providing means, a signal generating composition including a label itself or a label-conjugated oligonucleotide may be used. Various methods (e.g., TaqManTM probe method, molecular beacon method, etc.) are known for generating the signal indicating the presence of the target analyte using the signal generating composition.

Here, the term "signal" refers to a measurable output. In addition, the measured magnitude or change of the signal serves as an indicator that qualitatively or quantitatively indicates characteristics of the target analyte, specifically, the presence or absence of the target analyte in the sample. Here, examples of the indicator include, but are not limited to, fluorescence intensity, luminescence intensity, chemiluminescence intensity, bioluminescence intensity, phosphorescence intensity, charge transfer, voltage, current, power, energy, temperature, viscosity, light scattering, radioactivity intensity, reflectance, transmittance, and absorbance.

The term "dataset" refers to the result of the amplification reaction for the target analyte in the sample. Specifically, the dataset refers to data obtained from the amplification reaction for the target analyte in the sample or data processed from the corresponding data. The dataset obtained from the amplification reaction may include an amplification cycle.

Here, the term "cycle" refers to a unit of change in conditions in performing multiple measurements accompanied by a change in certain conditions. The change in the certain conditions refers to an increase or decrease in, for example, temperature, reaction time, number of reactions, concentration, pH, or number of times of replications of a measurement target (e.g., nucleic acid). Therefore, the cycle may be a time or a process cycle, a unit operation cycle, and a reproductive cycle.

More specifically, the term "cycle" means one unit of repetition when a reaction of a certain process is repeated or is repeated based on a certain time interval. For example, in the case of the nucleic acid amplification reaction, one cycle refers to a reaction that includes a denaturation step of a nucleic acid, an annealing step of a primer, and an extension step of a primer. In this case, the change in the certain conditions refers to the increase in the number of times of repetitions of the reaction and the repeating unit of a reaction including the series of steps is designated as one cycle.

Meanwhile, a dataset obtained from the amplification reaction includes multiple data points including cycles of the amplification reaction and signal values in the cycles.

Here, the term "signal value" refers to a value that is quantified according to a certain scale of a level of a signal (e.g., signal intensity) actually measured in the cycle of the amplification reaction, or a modified value thereof. The modified value may include a mathematically processed signal value of the actually measured signal value (i.e., a signal value of a raw dataset), and may include, for example, logarithmic values or derivatives.

The term "data point" refers to a single coordinate value that includes a cycle and a signal value. In addition, the term "data" refers to all information that constitutes the dataset. For example, each of the cycle and the signal value of an amplification reaction may correspond to data. The data points obtained from the amplification reaction may be represented as coordinate values that may be represented in a two-dimensional orthogonal coordinate system. In the coordinate values, an X-axis represents the corresponding cycle number, and a Y-axis represents the signal value measured or processed in the corresponding cycle.

The term "dataset" refers to a set of the data points. For example, the dataset may be the set of the data points obtained directly from the amplification reaction performed in the presence of the signal generating composition, or may be a dataset modified from such a dataset. The dataset may be a portion or all of the multiple data points obtained from the amplification reaction or the modified data points thereof.

The dataset may include a dataset obtained by processing multiple datasets. When analysis of multiple target analytes is performed in one reaction vessel, the datasets for the multiple target analytes may be obtained by processing the datasets obtained from the reaction performed in the one reaction vessel. For example, the datasets for the multiple target analytes performed in one reaction vessel may be obtained by processing the multiple datasets obtained from signals measured at different temperatures. For example, a method has been known in which signals generated at each of different detection temperatures are detected using a single type of detector to detect two target nucleic acid sequences in a sample (Reference: Korean Patent No. 10-2050601).

According to one implementation example, the dataset may be a raw dataset obtained from a detection device, a mathematically transformed dataset of the raw dataset, a normalized dataset of the raw dataset, or a normalized dataset of the mathematically transformed dataset.

Here, the raw dataset refers to a dataset including signal values directly obtained from the amplification reaction. For example, the raw dataset includes a set of signal values that is obtained from the detection device on which the amplification reaction for detecting the target analyte is performed, undergoes basic signal processing on the detection device, and then passes on to a signal analysis step.

In addition, the mathematically transformed dataset of the raw dataset refers to a dataset transformed from the raw dataset through mathematical processing. For example, the mathematically processed dataset may be a dataset obtained by removing at least some of background signals from the raw dataset, i.e., a baseline-subtracted dataset. The baseline-subtracted dataset may be obtained from various methods known in the art (e.g., U.S. Pat. No. 8,560,247). For another example, the mathematically processed dataset may be a dataset obtained by removing a noise signal from the raw dataset due to at least some of the background signals, noise, interference, etc.

In addition, the normalization refers to a process of reducing or eliminating a signal deviation between the datasets for multiple reactions. The normalization is an aspect of correction or adjustment that corrects or transforms data (especially, signal values) of the dataset for analysis purposes.

According to one embodiment, the dataset includes 200 or fewer, 150 or fewer, 100 or fewer, 50 or fewer, 40 or fewer, and 30 or fewer data points. According to one embodiment, the dataset includes 2 or more, 5 or more, 10 or more, and 20 or more data points. The dataset may be plotted, thereby obtaining an amplification curve.

The term "channel" refers to a means for detecting a single type of signal. In a detector comprising several channels (e.g., photodiodes) for detecting several different types of signals, each channel corresponds to a means for detecting a single type of signal. According to one embodiment, a single type of signal may be measured or detected by a single type of label. For example, detecting two or more targets in a single channel may refer to detecting signals generated at each of different detection temperatures together to detect two target nucleic acid sequences in a sample using a single type of label.

Among these, the label include a fluorescent label, a luminescent label, a chemiluminescent label, an electrochemical labels, and a metal label. The labels may be used as a label itself, such as an intercalating dye. Alternatively, the label is a form of a single label or an interactive dual label including a donor molecule and an acceptor molecule, and may be used in the form of bound to one or more oligonucleotides.

The term "cycle threshold (Ct)" may be interpreted as meaning the time for the intensity of the signal value in the amplification curve in the dataset representing the result of the amplification reaction to reach a predetermined threshold or the cycle number expressed by the number of times of reactions, but the calculation scheme of the Ct or the meaning of the Ct is not limited thereto. According to one embodiment, the Ct may be broadly interpreted as a meaning encompassing a signal value, a cycle value, a measurement value of a specific parameter, etc., when predetermined analysis results (e.g., a derivative for the amplification curve, etc.) derived from the dataset satisfy a predetermined condition. According to one embodiment, the Ct may be interpreted as a meaning indicated by the terms cross point ($C_P$), take-off point (TOP), or quantification cycle ($C_Q$) used in the present technical field.

The term "quantitative value" indicates a quantitation result. According to one embodiment, the quantitative value of the target analyte is a result derived by absolutely quantitating the result of the amplification reaction of the target analyte. For example, the quantitative value may refer to an absolute quantitative value for an initial concentration of the target analyte present in the corresponding sample. As an example of the absolute quantitation technique, there are the above-described standard curve technique, digital PCR technique, etc. According to another embodiment, the quantitative value of the target analyte is a result derived by relatively quantitating the result of the amplification reaction of the target analyte. For example, the quantitative value may refer to a relative quantitative value for an initial concentration of the target analyte present in the corresponding sample. As an example of the relative quantitation technique, there are the above-described standard curve technique, comparative CT technique, etc.

Meanwhile, in the present disclosure, estimating the Ct or the quantitative value refers to estimating the Ct or the quantitative value understood as the above-described meaning using an estimation method according to one embodiment of the present disclosure, and is not limited to a typical calculation scheme implied by the term Ct or the quantitative value itself.

In the present specification, the oligonucleotide refers to a natural or modified monomer or linear oligomer of linkages, and may include deoxyribonucleotides and ribonucleotides, and may hybridize specifically to a target nucleic acid sequence and may be naturally occurring or artificially synthesized. The oligonucleotide of the present invention may include naturally occurring dNMP (i.e., dAMP, dGMP, dCMP, and dTMP), nucleotide analogues or derivatives. In particular, the oligonucleotide is a single strand composed of deoxyribonucleotides. The oligonucleotide includes an oligonucleotide that hybridizes with a cleavage fragment that occurs dependently on the target nucleic acid sequence. Specifically, the oligonucleotide includes a primer and/or a probe.

In the present specification, the term "primer" refers to an oligonucleotide that may act as an initiation point of synthesis under conditions that induce a synthesis of a primer extension product complementary to a nucleic acid strand (template), i.e., the presence of nucleotides and a polymerization agent such as DNA polymerase, and conditions of suitable temperature and pH.

In the present specification, the term "probe" refers to a single-stranded nucleic acid molecule that includes a site or sites complementary to the target nucleic acid sequence. In addition, the probe may include a label that may generate a signal for target detection.

FIG. 1 schematically illustrates a block diagram of a computer device 1000 according to an embodiment.

Referring to FIG. 1, the computer device 1000 may include a memory 100, a communication unit 200, and a processor 300. The configuration of the computer device 1000 shown in FIG. 1 is merely an example presented in a simplified form. In one embodiment, the computer device 1000 may include additional components for implementing the computing environment of the computer device 1000, and only some of the illustrated components may be included in the computer device 1000.

The computer device 1000 may refer to a node constituting a system for implementing embodiments of the present disclosure. The computer device 1000 may include any type of user terminal and/or any type of server.

The user terminal may include any type of terminal capable of interacting with a server or other computing device. For example, the user terminal may include a mobile phone, smartphone, laptop computer, personal digital assistant (PDA), slate PC, tablet PC, or ultrabook.

The server may include any type of computing system or computing device, such as a microprocessor, mainframe computer, digital processor, portable device, or device controller. In one embodiment, the server may include an entity that stores and manages a plurality of datasets. The server may include a storage unit (not shown) for storing training datasets for training an estimation model to be described later. The storage unit may be included within the server or may exist under the control of the server. In another example, the storage unit may exist outside the server and be implemented in a form capable of communicating with the server. In this case, the storage unit may be managed and controlled by another external server different from the server.

A computer device 1000 may perform technical features according to embodiments to be described below. For example, the computer device 1000 may estimate molecular diagnostic analysis results for the target analyte in the sample using the dataset representing the result of the amplification reaction for the target analyte in the corresponding sample.

Here, the molecular diagnostic analysis results are analysis results of a molecular diagnostic test performed on a sample that includes or may include the target analyte, for example, the molecular diagnostic analysis results comprehensively refer to analysis results that can be derived from a dataset obtained from a nucleic acid amplification reaction (e.g., PCR) using a sample collected from a test subject. The molecular diagnostic analysis results according to one embodiment may include an assessment result that qualitatively or quantitatively determines the target analyte in the sample and/or an assessment basis that may be used in the assessment process. For example, the assessment result may include a positive/negative determination result for the target analyte in the sample for the presence or absence of the target analyte in the sample, an absolute quantitative value or a relative quantitative value for the amount (e.g., initial concentration) of target analyte, etc. In addition, the assessment basis includes one or more signal values, measurement values, or computation values, such as the Ct, that can be used as positive/negative assessment criteria or quantitation criteria.

According to one embodiment, the molecular diagnostic analysis results may include at least any one selected from the group consisting of the Ct, the quantitative value, the positive/negative determination result for the target analyte in the sample, and the suitability assessment result of oligonucleotide candidates. As described above, the Ct refers to a Ct in an amplification curve in the corresponding dataset. The quantitative value refers to the quantitative value of the target analyte in the sample from which the corresponding dataset is obtained. The positive/negative determination result for the target analyte in the sample represents the presence or absence (e.g., positive (+) when present, negative (-) when absent) of the target analyte in the sample from which the corresponding dataset is obtained. The suitability assessment result of oligonucleotide candidates is the suitability assessment result of oligonucleotide candidates to be used in the corresponding amplification reaction, and indicates whether the oligonucleotide (e.g., the primer, the probe) included in the sample from which the corresponding dataset is obtained is suitable as the oligonucleotide candidates for detection of the target analyte included in the corresponding sample, or the degree of suitability. For example, when it is assessed to be suitable as the oligonucleotide candidate, the sequence information of the corresponding oligonucleotide may be applied to a molecular diagnostic reagent used for detecting the corresponding target analyte. According to one embodiment, the molecular diagnostic analysis results may include the Ct, the quantitative value, the positive/negative determination result for the target analyte in the sample, or the suitability assessment result of oligonucleotide candidates, and in another embodiment, may include some combinations of the Ct, the quantitative value, the positive/negative determination result for the target analyte in the sample, and the suitability assessment result of oligonucleotide candidates. The description of the molecular diagnostic analysis results described above is only an example, and the molecular diagnostic analysis results in the present disclosure are not limited thereto.

The memory 100 may store at least one instruction executable by the processor 300. In one embodiment, the memory 100 may store any type of information generated or determined by the processor 300, and any type of information received by the computer device 1000. In one embodiment, the memory 100 may be a storage medium that stores computer software which allows the processor 300 to perform operations according to embodiments of the present disclosure. Accordingly, the memory 100 may refer to computer-readable storage medium for storing software code required to perform embodiments of the present disclosure, data to be processed by the code, and results of code execution.

According to an embodiment, the memory 100 may refer to any type of storage medium. For example, the memory 100 may include at least one type of storage medium such as a flash memory, hard disk, multimedia card micro type, card-type memory (e.g., SD or XD memory), RAM (Random Access Memory), SRAM (Static Random Access Memory), ROM (Read-Only Memory), EEPROM (Electrically Erasable Programmable Read-Only Memory), PROM (Programmable Read-Only Memory), magnetic memory, magnetic disk, or optical disk. The computer device 1000 may also operate in connection with web storage that performs the storage function of the memory 100 over the Internet. The above description of the memory is merely an example, and the memory 100 of the present disclosure is not limited thereto.

The communication unit 200 may be configured regardless of its communication type, such as wired or wireless, and may be implemented using various communication networks such as a personal area network (PAN) or a wide area network (WAN). The communication unit 200 may also operate based on the well-known World Wide Web and may utilize short-range wireless transmission technologies such as infrared (IrDA: Infrared Data Association) or Bluetooth. For example, the communication unit 200 may be responsible for transmitting and receiving data necessary for performing a technique according to an embodiment of the present disclosure.

The processor 300 may execute at least one instruction stored in the memory 100 to perform the technical features according to embodiments to be described later. In one embodiment, the processor 300 may include at least one core and may include processors for data analysis and/or processing, such as a CPU (central processing unit), GPGPU (general purpose graphics processing unit), or TPU (tensor processing unit) of the computer device 1000.

The processor 300 according to an embodiment may perform operations for training a neural network. The processor 300 may perform computations for neural network training, such as processing input data for training in deep learning, extracting features from input data, computing errors, and updating the weights of the neural network using backpropagation. At least one of the CPU, GPGPU, or TPU of the processor 300 may process training of a network function. For example, the CPU and GPGPU may together process the training of a network function and classification of data using the network function. In addition, in one embodiment of the present disclosure, the training and classification may be performed using processors of multiple computing devices in combination. Furthermore, the computer program executed on the computing device according to an embodiment of the present disclosure may be executable by the CPU, GPGPU, or TPU.

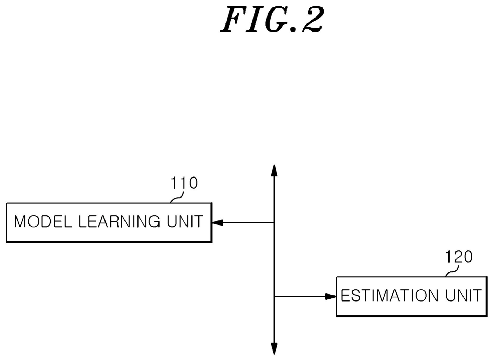

Various technical features performed by the processor 300 will be described below with reference to FIG. 2.

FIG. 2 illustrates a modular representation of software implemented by the computer device 1000 shown in FIG. 1. Referring to FIG. 2, the software, which is implemented as the hardware processor 300 executes at least one instruction stored in the memory 100, may be modularized into at least one of a model learning unit 110 and an estimation unit 120. For example, the model learning unit 110 and the estimation unit 120 may each be implemented as computer programs, and instructions and data for their execution may be stored in the memory 100 and executed by the processor 300. However, this is not limited thereto. According to one embodiment, the instructions and data for executing the model learning unit 110 may be stored in the memory of a server among a plurality of entities constituting the computer device 1000 and executed on the server, while the instructions and data for executing the estimation unit 120 may be stored in the memory of a user terminal among the plurality of entities and executed on the user terminal.

According to one embodiment of the present disclosure, a model learning unit 110 may be implemented to obtain the estimation model trained to estimate the molecular diagnostic analysis results for the target analyte in the sample. Specifically, the model learning unit 110 may obtain multiple training datasets for model training, and train the estimation model using the multiple training datasets. According to one embodiment, the estimation model may include an artificial intelligence-based model.

In the present specification, the estimation model may refer to any type of computer program operating based on a network function, artificial neural network, and/or neural network. Throughout the present specification, the terms "model," "neural network," "network function," and "neural network" may be used interchangeably. A neural network consists of one or more nodes interconnected through one or more links, forming relationships between input and output nodes within the network. The characteristics of the neural network may be determined based on the number of nodes and links, the relationships among them, and the weights assigned to each link. In one embodiment, the neural network may be of any well-known type.

The estimation model according to an embodiment may include at least one of a convolutional neural network (CNN), deep neural network (DNN), recurrent neural network (RNN), vision transformer (ViT), and generative adversarial network (GAN). Depending on the embodiment, a DNN may be interpreted broadly as a neural network including multiple hidden layers in addition to the input and output layers, and may encompass CNNs, RNNs, autoencoders, GANs, deep belief networks (DBNs), and transformers. The description of the estimation models above is merely exemplary and is not intended to limit the scope of the present disclosure.

The estimation model according to an embodiment may be trained using at least one of supervised learning, unsupervised learning, semi-supervised learning, self-supervised learning, and reinforcement learning. The training may refer to a process in which a deep learning model applies knowledge to the neural network in order to perform a specific task.

The estimation model according to an embodiment may be trained to minimize output error. The training process may include repeatedly inputting training data to the estimation model, calculating the error between the model's output and the target, and updating the weights of each node by backpropagating the error from the output layer toward the input layer to reduce the error. In supervised learning, labeled data is used in which each training sample has an associated correct answer, whereas in unsupervised learning, unlabeled data is used. The amount of change in each node's weight may be determined by a learning rate. The forward computation of the input data through the neural network and the backpropagation of the error together may form one training cycle (epoch). The learning rate may be variably applied depending on the number of epochs.

According to one embodiment, the estimation model may be trained to output an estimation result for the corresponding molecular diagnostic analysis results when receiving a shape similarity for each reference pattern. Here, the shape similarity indicates the degree to which the shape of the target curve generated based on the dataset for which the molecular diagnostic analysis results are to be estimated is similar to that of a pre-established reference pattern. For example, as the target curve obtained by plotting the dataset becomes more similar to a shape of a reference pattern following a predetermined signal pattern, the shape similarity of the target curve with respect to the corresponding reference pattern may be quantified as a large value. A specific embodiment in which the estimation model is trained will be described later.

According to one embodiment of the present disclosure, an estimation unit 120 may be implemented to estimate the molecular diagnostic analysis results including at least any one selected from the group consisting of the Ct in the corresponding target curve, the quantitative value of the target analyte in the sample, the positive/negative determination result for the target analyte in the sample, and the suitability assessment result of oligonucleotide candidates to be used for the corresponding amplification reaction from the dataset for estimating the molecular diagnostic analysis results using the trained estimation model. Specifically, the estimation unit 120 may obtain the dataset representing the result of the amplification reaction for the target analyte in the sample, calculate the shape similarity for each reference pattern by comparing the target curve generated based on the corresponding dataset to each of the multiple pre-established reference patterns, and provide the calculated shape similarity for each reference pattern to the estimation model to obtain the molecular diagnostic analysis results from the estimation model.

In the process of training the model and the process of estimating the molecular diagnostic analysis results, a process of preprocessing a scheme for calculating the shape similarity for each reference pattern using the dataset and multiple reference patterns may be performed. Here, the preprocessing process may include a series of processes for extracting the degree of similarity between the shape of the target curve generated based on the dataset and the shapes of each of various reference patterns as a kind of feature, processing the extracted feature as preprocessing data, and providing the preprocessing data including such features as input to the estimation model. For example, the preprocessing process may be performed to calculate the shape similarity for each reference pattern by comparing the amplification curve obtained by plotting the dataset to each of the pre-established reference patterns with various shapes and process the calculated shape similarity as an input to the estimation model. In the estimation model, a process of extracting features for the preprocessed shape similarity for each reference pattern and estimating the molecular diagnostic analysis results may be performed.

The preprocessing process is different from the conventional preprocessing scheme that simply transforms the dataset into a format suitable for computation in a deep learning model. Conventionally, the analysis target, such as the dataset, is preprocessed by a scheme that simply transforms an analysis target into an image or a vector and provided to the model. Accordingly, since the deep learning model performs feature extraction from relatively fragmentary preprocessing data, there was a limitation that estimation performance was not sufficiently secured.

However, according to one embodiment of the present disclosure, the preprocessing process is performed by a scheme in which a feature representing the "shape similarity with various reference patterns" of the analysis target, such as the dataset, is extracted in advance and transformed into a feature map, and the deep learning model performs feature extraction from the feature map that includes such features. Therefore, the data input to the deep learning model is advanced by the preprocessing process, and it becomes easy to extract meaningful features from the deep learning model, thereby improving the estimation performance.

Various embodiments regarding the operations/functions of the model learning unit 110 and estimation unit 120 presented above will be described in more detail below.

Preprocessing process for training

The model learning unit 110 may prepare training of the estimation model through a preprocessing process for training. The preprocessing process for training according to one embodiment may include a process of preprocessing a dataset for training as input data to be provided to the estimation model and a process of obtaining multiple training datasets.

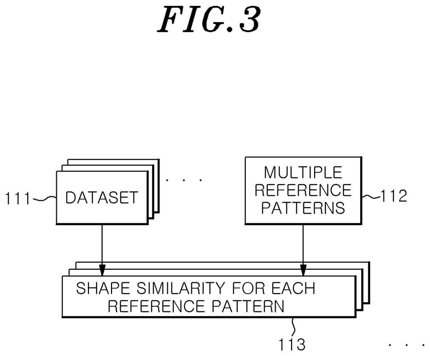

FIG. 3 exemplarily illustrates a conceptual diagram of the process of preprocessing the dataset for training as the input data to be provided to the estimation model according to one embodiment.

Referring to FIG. 3, the model learning unit 110 may obtain multiple datasets 111 and multiple reference patterns 112 for training, respectively. According to one embodiment, the model learning unit 110 may obtain the multiple datasets 111 and/or the multiple reference patterns 112 from a memory 100 or a storage device by the processor 300 or from another external device through a communication unit 200.

Here, the multiple datasets 111 represent results of multiple amplification reactions for the target analytes in multiple samples. According to one embodiment, each dataset 111 may represent the results of the amplification reaction performed targeting one type of target analyte detectable in a single channel in one sample. For example, the amplification curve in the corresponding dataset 111 may be represented in a shape in which a signal (e.g., a sigmoid curve) dependent on the presence of the corresponding target analyte and a signal (e.g., a background signal, noise, interference, etc.) independent on the presence of the corresponding target analyte overlap each other. According to another embodiment, each dataset 111 may represent the results of the amplification reactions performed targeting two or more types of target analytes detectable in a single channel in one sample. For example, the amplification curve in the corresponding dataset 111 may be represented in a shape in which a signal (e.g., the first sigmoid curve) dependent on the presence of a first target analyte, a signal (e.g., the second sigmoid curve) dependent on the presence of a second target analyte, and the signal (e.g., background signal, noise, interference, etc.) independent on the presence of the target analyte overlap each other. According to another embodiment, each dataset 111 may refer to a dataset for one type of target analyte extracted from the results of the amplification reactions performed targeting two or more types of target analytes described above.

The following description focuses on the former embodiment, but is not limited thereto.

According to one embodiment, each dataset 111 may be obtained from the amplification reaction using a sample collected from a test subject to detect the presence or absence of the target analyte. For example, in a process (e.g., a test process using a pre-developed molecular diagnostic reagent) of analyzing results of a nucleic acid amplification reaction using a pre-designed oligonucleotide, the corresponding oligonucleotide (e.g., a primer) may be accommodated in the sample collected from the test subject (e.g., a patient), and the dataset 111 may be obtained from performing the amplification reaction on an unknown sample in which the presence or absence of the target analyte is unknown.

According to another embodiment, each dataset 111 may be obtained from the amplification reaction using oligonucleotide candidates designed for detection of the target analyte. For example, in the process (e.g., the process of developing the molecular diagnostic reagent) of designing oligonucleotide for the detection of the target analyte, the oligonucleotide candidates (e.g., primer) designed to be used for the detection of the corresponding target analyte are accommodated in the sample together with the corresponding target analyte, and the dataset 111 may be obtained by performing the amplification reaction on the sample for which the presence or absence of the target analyte is known. For example, when the corresponding primer includes a sequence specific to the corresponding target analyte, an appropriate amplification curve shape is expected to appear in the dataset 111 as a result of the nucleic acid amplification. As another example, when the corresponding primer does not include the sequence specific to the corresponding target analyte, or even if the primer includes a specific sequence, when the primer reacts with another composition (e.g., another primer) and fails to generate the signal specific to the corresponding target analyte, the appropriate amplification curve shape is not expected to appear in the corresponding dataset 111 as a result of the nucleic acid amplification. In the above-described multiple datasets 111, the nucleic acid sequences of the oligonucleotide candidates may be the same or different, and the target analytes targeted by each oligonucleotide candidate may also be the same or different.

According to one embodiment, the multiple datasets 111 may include signal values at each multiple cycle obtained as a result of the amplification reaction, or results of an n-th derivative of curves connecting the signal values at each multiple cycle. Here, n is a natural number, and may be, for example, a natural number greater than or equal to 1 and less than or equal to 3.

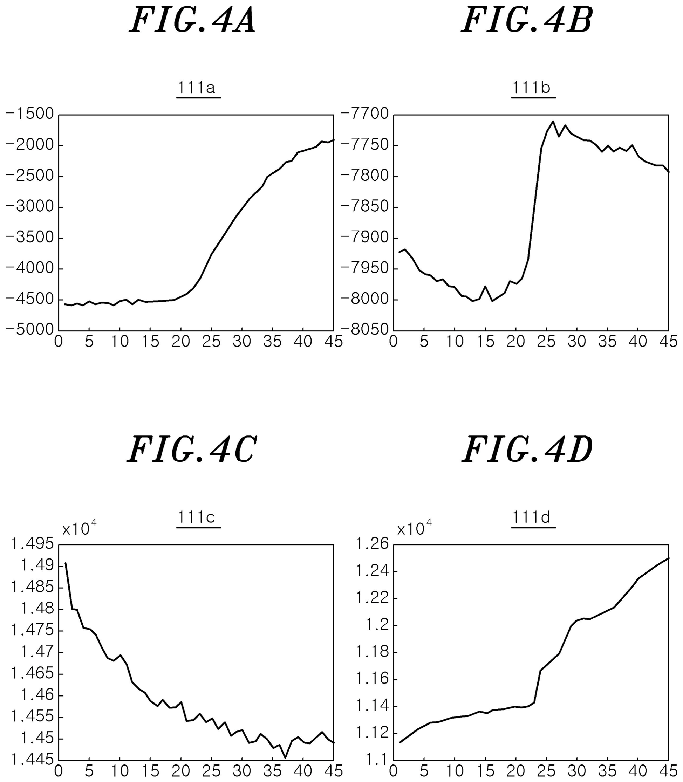

FIG. 4 exemplarily illustrates the multiple datasets 111 according to one embodiment.

Referring to FIG. 4, each dataset 111 according to one embodiment may include the signal values in each multiple cycle. For example, each dataset 111 may be a set of coordinate values including the cycles and the signal values, and may be represented as coordinate values on a two-dimensional orthogonal coordinate system. In the coordinate values, the X-axis may represent the cycle number, and the Y-axis may represent the signal value (e.g., relative fluorescence units (RFU)) measured or processed in the corresponding cycle.

According to one embodiment, the target curve generated based on each dataset 111 may refer to a line that connects a set of the corresponding coordinate values by plotting the set of the corresponding coordinate values on the two-dimensional orthogonal coordinate system. According to another embodiment, the target curve generated based on each dataset 111 may refer to an approximate function that approximates the set of the corresponding coordinate values. For example, the target curve may be approximated in the form of a predetermined function that represents a linear correlation between a dependent variable y (e.g., signal value in each cycle) and an independent variable x (e.g., the corresponding cycle number) through linear regression analysis on the set of the corresponding coordinate values.

As illustrated in FIG. 4, each dataset 111 may appear in various shapes, and the target curve generated based on each dataset 111 may be amplified in different shapes by various factors that affect each amplification reaction. The above-described various factors may include the presence or absence of the target analyte in the sample, the relative or absolute amount of the target analyte in the sample, the number of types of the target analyte to be detected in a single channel, the type or size causing the background signal or the abnormal signal, etc.

A first dataset 111a is an example of a normal positive case, and illustrates an amplification curve when the target analyte is present in the sample. A second dataset 111b is an example of an abnormal negative case, and illustrates an amplification curve when the target analyte is absent in the sample and an abnormal signal with a discretely increasing magnitude of amplitude is included. A third dataset 111c is an example of a normal negative case, and illustrates an amplification curve when the target analyte is absent in the sample. A fourth dataset 111d is another example of the abnormal negative case, and illustrates an amplification curve when the target analyte is absent in the sample and the abnormal signal with a discrete increasing magnitude of amplitude is included. According to one embodiment, when each dataset 111 represents the result of the amplification reaction performed targeting two or more types of target analytes as described above, the amplification curve may appear as a composite shape in which two or more of these positive or negative cases are mixed.

Each dataset 111 according to another embodiment may include an nth derivative result for a curve connecting signal values in each multiple cycle. For example, the nth derivative result for the corresponding curve may be a first derivative result, a second derivative result, or a third derivative result for the corresponding curve. For example, each dataset 111 may include a first derivative derived by differentiating an approximation function that approximates the set of the coordinate values including the cycles and the signal values, or a set of change amount coordinate values derived by using a change amount of a dependent variable value and a change amount of an independent variable value within the set of the coordinate values. According to the embodiment, each dataset 111 may include the second derivative result, a third or higher derivative result, or an integration result, etc., for a curve connecting the signal values in each multiple cycle. The following describes the subsequent processes centered on one embodiment in which the dataset includes the signal values in each multiple cycle, but is not limited thereto.

According to one embodiment, the model learning unit 110 may receive the multiple datasets 111 from the storage unit included in a server. For example, the storage unit implemented as a database may store and manage the datasets collected from the detection device that has performed the amplification reaction or a signal analysis device that performs signal analysis on the result of the amplification reaction. According to another embodiment, the model learning unit 110 may read the multiple datasets 111 from a storage device (e.g., USB (Universal Serial Bus)) through a data input port.

Meanwhile, the multiple reference patterns 112 refer to patterns of various different signal shapes that mainly appear as a result of the amplification reaction. According to one embodiment, the multiple reference patterns 112 may be in the form of a predetermined function or the set of the coordinate values having various representative shapes of amplification curves shown in various experimental data for each case in which a target analyte is present or is absent in a sample, and may be plotted as a curve by the corresponding function or a line connecting the corresponding coordinate values on the two-dimensional orthogonal coordinate system. For example, when the dataset 111 is the set of the coordinate values including the cycles and the signal values, each reference pattern 112 may be in the form of a predetermined function representing a correlation between a cycle number x, which is an independent variable, and a reference signal value y, which is a dependent variable, in each cycle. The reference signal value represents a value that is referenced by comparing with the signal value in each cycle.

According to one embodiment, the multiple reference patterns 112 may be determined in different shapes by considering various factors affecting the nucleic acid amplification reaction. Specifically, the multiple reference patterns 112 may be determined based on at least any one selected from the group consisting of a first amplification reference pattern in a case where the target analyte is absent in the sample, a second amplification reference pattern in cases where one type of target analyte detectable in a single channel is present at a relatively high concentration or a relatively low concentration in the corresponding sample, respectively, a third amplification reference pattern in cases where two or more types of target analytes detectable in the single channel are present at the same concentration or at different concentrations in the corresponding sample, respectively, an aspect of a background signal included in a result of the amplification reaction, an aspect of an abnormal signal included in the result of the amplification reaction, and an aspect of a non-specific signal due to an amplification reaction other than an intended amplification.

According to one embodiment, the first amplification reference pattern may include a reference pattern in at least one of the cases where the magnitude of the amplitude of the target curve is constant, where the amplitude change amount of the target curve varies linearly within a predetermined range corresponding to the absence of the target analyte, and where the amplitude change amount of the target curve is within a predetermined range corresponding to the absence of the target analyte and the magnitude of amplitude varies along an exponential region and a plateau region. For example, as the amplitude range of the target curve, there may be a constant less than or equal to a predetermined set value, a linear function whose slope is a negative (-) value, a growth curve-like function (e.g., sigmoid, logistic, Gompertz, Chapman), etc.

According to one embodiment, the second amplification reference pattern may include a reference pattern in the case where the amplitude change amount of the target curve is within a predetermined range corresponding to the presence of the target analyte and the magnitude of amplitude varies along the exponential region and the plateau region. According to one embodiment, among the second amplification reference patterns, a 2-1th amplification reference pattern in the case where the corresponding target analyte is present at a relatively high concentration may have a relatively small cycle number in the exponential region, and a 2-2th amplification reference pattern in the case where the target analyte is present at a relatively low concentration may have a relatively large cycle number in the exponential region. For example, there may be the growth curve-like function in which the amplitude range of the target curve is greater than or equal to a predetermined set value, and the cycle number at an inflection point has a relative high set value or a relative low set value depending on the relative high concentration or the relative low concentration of the target analyte.

According to one embodiment, the third amplification reference pattern may include a reference pattern in the case where two or more second amplification reference patterns overlap each other. According to one embodiment, a 3-1th amplification reference pattern in the case where two or more types of target analytes are present at the same concentration in the corresponding sample may include a reference pattern obtained by overlapping two or more 2-1th amplification reference patterns or a reference pattern obtained by adding two or more 2-2th amplification reference patterns. According to one embodiment, the 3-2th amplification reference pattern in the case where two or more types of target analytes are present at different concentrations in the corresponding sample may include a reference pattern obtained by adding two or more of multiple 2-1th amplification reference patterns and multiple 2-2th amplification reference patterns having different set values for the cycle number at the inflection point.

According to one embodiment, the first reference pattern by the aspect of the background signal included in the result of the amplification reaction may include a reference pattern in at least one of the cases where the magnitude of amplitude included in the result of the amplification reaction decreases exponentially, where the magnitude of amplitude included in the result of the amplification reaction decreases linearly, and where the magnitude of amplitude included in the result of the amplification reaction maintains a predetermined value. Examples of the first reference pattern may include a constant, a linear function whose slope has a negative (-) value, an exponential decay function, etc.

According to one embodiment, the first reference pattern may be a pattern representing the background signal obtained as a result of the amplification reaction when the amplification reaction is performed on the sample in which the target analyte is absent. According to one embodiment, the aspect of the background signal may depend on at least one of the type of the detection device (e.g., CFX96) in which the amplification reaction is performed, the type of the reaction vessel (e.g., the shape of the vessel, the accommodation scheme, etc.) in which the sample is accommodated, the type of the plate (e.g., 12×8 layout) in which the reaction vessel is accommodated, and whether the reaction vessel is closed. The reference pattern by the aspect of the background signal may be determined by considering the above-described matters.

According to one embodiment, the second reference pattern according to the aspect of the abnormal signal included in the result of the amplification reaction may include a reference pattern in at least one of the case in which the magnitude of amplitude included in the result of the amplification reaction increases discretely, in the case of receiving signal interference (e.g., cross-talk) from another channel, in the case in which the magnitude of amplitude included in the result of the amplification reaction increases linearly, and in the case in which white noise is received from the outside. For example, the reference pattern may be in the form of a step function in which the amplitude jumps, a nonlinear function in which the amplitude shifts on the Y-axis, a linear function whose slope is a positive (+) value, a quadratic function protruding upward or downward, a white noise function following a standard normal distribution, etc.

According to one embodiment, the second reference pattern may be a pattern representing noise, interference, etc., caused by environmental causes. According to one embodiment, the aspect of the abnormal signal may depend on at least one of the number and type of channels, the number of target analytes to be detected in the single channel, and a signal change aspect in an adjacent reaction vessel. In addition, the reference pattern according to the aspect of the abnormal signal may be determined in consideration of the above-described matters.

According to one embodiment, the third reference pattern according to the aspect of the non-specific signal (non-specific signal from other amplification reactions than desired amplification) due to the amplification reactions other than the intended amplification may include a reference pattern in the case where the magnitude of amplitude included in the result of the amplification reaction changes according to the exponential region and the plateau region, but may include a reference pattern in the case where a weak amplification reaction is present since the amplitude change amount does not reach a predetermined range corresponding to the presence of the target analyte. For example, the corresponding reference pattern may be in the form of the growth curve-like function (e.g., sigmoid, logistic, Gompertz, Chapman), etc., in which the amplitude range is less than or equal to a predetermined setting value.

According to one embodiment, the third reference pattern may be a pattern representing the case where the weak amplification signal is detected even if the amplification reaction is performed on the sample in which the target analyte is absent and thus no signal specific to the target analyte is expected or intended to occur. For example, the third reference pattern may be a pattern representing the case where the oligonucleotide (e.g., the primer) for detecting the target analyte reacts with another composition (e.g., another primer, a probe, etc.) contained in the corresponding sample, thereby causing the weak amplification reaction. Similarly, the aspect of such a non-specific signal may depend on at least one of the number and type of channels, the number of target analytes to be detected in the single channel, and the number of oligonucleotides having different sequences used to detect one target analyte, and may be determined in consideration of the above-described matters.

According to one embodiment, the multiple reference patterns 112 may be determined to have the shape in which the reference patterns in at least some of the above-described cases overlap each other or a shape modified therefrom.

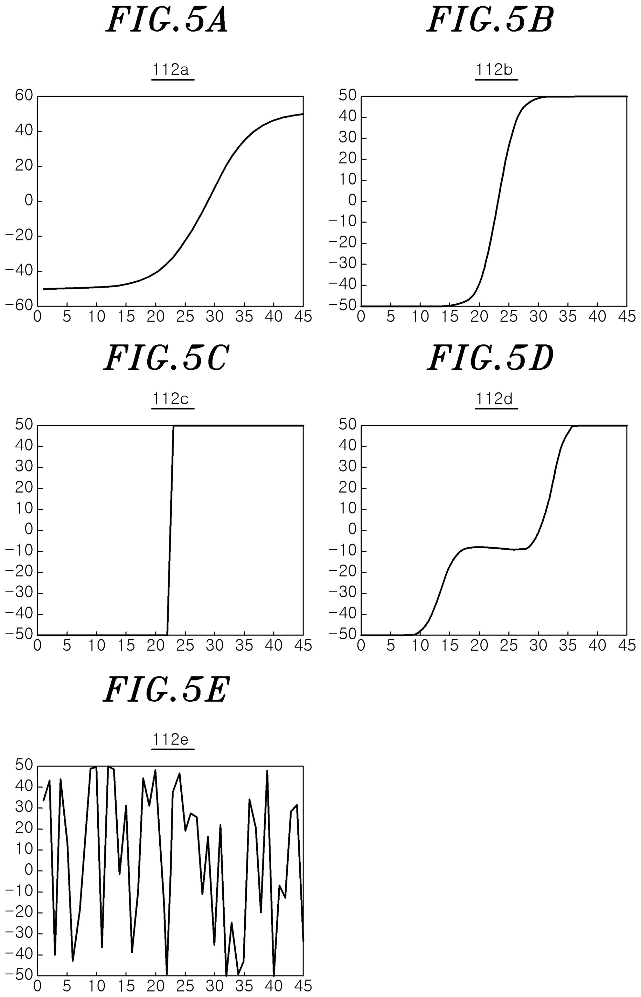

FIG. 5 exemplarily illustrates the multiple reference patterns 112 according to one embodiment. As illustrated in FIG. 5, each reference pattern 112 may be modeled to have various shapes. The first reference pattern 112a exemplifies the second amplification reference pattern with the growth curve shape by the sigmoid function. A second reference pattern 112b exemplifies the second amplification reference pattern with the growth curve shape by the sigmoid function in which the value of the parameter representing the slope is relatively large. A third reference pattern 112c exemplifies the second reference pattern with the shape by the step function. A fourth reference pattern 112d exemplifies a 3-2th amplification reference pattern with the shape in which the growth curve shapes by two sigmoid functions with different cycle number at the inflection points are continuous. A fifth reference pattern 112e exemplifies the second reference pattern with the shape in which the value changes irregularly by a white noise function.

According to one embodiment, the model learning unit 110 may read the multiple pre-established reference patterns 112 from the memory 100 or receive the multiple pre-established reference patterns 112 from another device. According to another embodiment, the model learning unit 110 may determine the multiple reference patterns 112 using the multiple datasets representing the results of the multiple amplification reactions. For example, the model learning unit 110 may perform cluster analysis on the multiple datasets 111 collected in the storage unit of the server to group datasets showing relatively similar curve shapes or signal change patterns into multiple groups, and may model reference patterns to have shapes representing each group. According to one embodiment, the multiple reference patterns 112 may be updated at a preset cycle or by a user request.

The description of the multiple datasets 111 and the multiple reference patterns 112 described above is only an example, and the present disclosure is not limited thereto. The shapes of lines, value ranges, scales, etc., illustrated in the drawings are only examples, and may differ depending on design matters.

Referring back to FIG. 3, the model learning unit 110 may calculate a shape similarity for each reference pattern 113 by comparing the target curve generated based on the dataset 111 to each of the multiple reference patterns 112. As described above, the shape similarity refers to a numerical representation of how similar the shapes of the comparison targets are. For example, the model learning unit 110 may quantifies the shape similarity by computing the degree of similarity not only in aspects such as the shape of the signal value changing according to the cycles among the comparison targets but also in aspects such as the magnitude of amplitude, the ratio of change amount, or the absolute value.

According to one embodiment, the shape similarity may be measured by computing a cross correlation between the target curve generated based on the dataset 111 and the reference pattern 112.

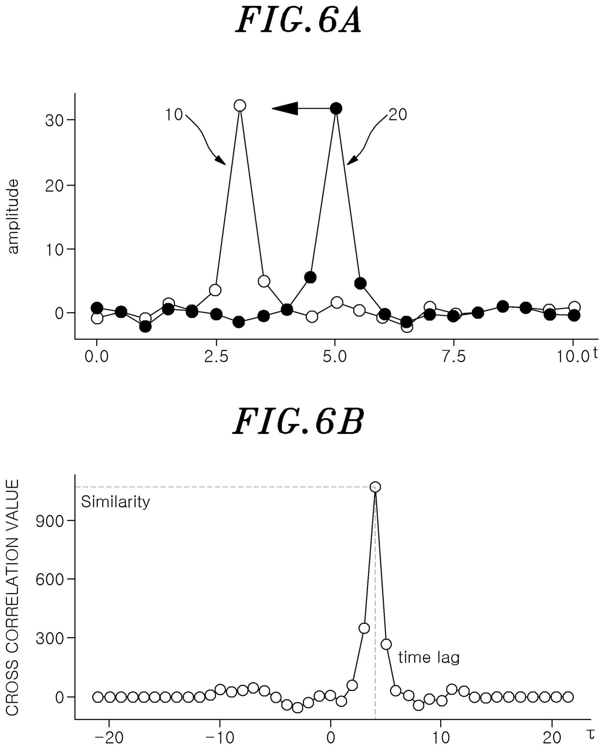

FIG. 6 exemplarily illustrates a process of measuring the shape similarity through the computation of the cross correlation according to one embodiment.

FIG. 6A illustrates a graph in which two comparison targets, a first signal 10 and a second signal 20, are displayed on a two-dimensional orthogonal coordinate system. The first signal 10 may correspond to one of the target curve generated based on the dataset 111 and the reference pattern 112, and the second signal 20 may correspond to the other. The model learning unit 110 may measure the shape similarity by computing the cross correlation for each shift amount when shifting any one of the first signal 10 and the second signal 20 by changing the shift amount of the first signal 10 and the second signal 20. The process of computing the cross correlation according to one embodiment may be understood with reference to the following Equation 1.

[Equation 1]

$$f \star g(\tau) = \int_{t=-\infty}^{t=\infty} f(t)g(t+\tau)dt$$

**Here,** t denotes the cycle number, and $\tau$ is the shift amount and denotes time displacement or cycle displacement. In addition, f(t) denotes the first function 10 where t is an independent variable, g(t) denotes the second function 20 where t is an independent variable, and g(t + $\tau$) denotes the independent variable t of the second function 20 shifted by $\tau$. In addition, f$\star$g($\tau$) denotes the result of computing the cross correlation between f(t) and g(t) by applying $\tau$ to g(t). Referring to Equation 1, the cross correlation value may be computed for each value of $\tau$ by changing the value of $\tau$ for g(t).

For example, after shifting the independent variable t of the second signal 20 by predetermined $\tau$, the cross correlation value when the corresponding $\tau$ is applied may be obtained by a scheme of summing the products of the dependent variable values of the first signal 10 and the second signal 20 at each time point when t changes from -∞ to ∞. For example, when $\tau$ = 0, by changing t from -∞ to ∞, the product between f(t) and g(t) may be obtained and added to measure ($\tau$, cross correlation value) = (0, 5). For another example, when $\tau$ = 3, by changing t from -∞ to ∞, the product between f(t) and g(t + 3) may be obtained and added to measure ($\tau$, cross correlation value) = (3, 960). In this way, the shape similarity between two signals 10 and 20 may be measured as the cross correlation values for each $\tau$ by changing the value of $\tau$ within a predetermined range (e.g., -∞ to ∞, -45 to +45, etc.).

FIG. 6B illustrates a graph that displays the shape similarity between two signals 10 and 20 on the two-dimensional orthogonal coordinate system. As described above, the shape similarity is the cross correlation values for each $\tau$ and may include the set of the coordinate values where the X-axis is $\tau$ and the Y-axis is the cross correlation value.

This shape similarity may include quantitative information about the degree to which the shapes are similar to each other regardless of the cycle number in which the values of each signal increase. As in FIG. 6A, even if the positions where the signal values of the two signals 10 and 20 change from each other are different, the two signals 10 and 20 may overlap each other as one signal shifts. As the shapes of the two signals 10 and 20 become more similar at the time point when the two signals 10 and 20 overlap each other, the magnitude of the product of the values of the two signals 10 and 20 increases. Therefore, the cross correlation may be used as a meaningful indicator to quantify the degree to which the signal shapes are similar regardless of the position.

In addition, the shape similarity may further include qualitative information about the cycle number in which the shapes appear similarly or the range thereof. As in FIG. 6B, the shape similarity includes the $\tau$ value (e.g., 3) at the time point (e.g., when the cross correlation value is the largest) when the shape is most similar, the range of $\tau$ (e.g., the cross correlation value is greater than or equal to a predetermined threshold (e.g., 300)) (e.g., 2 to 5) in which the shape is significantly similar, the pattern (e.g., whether the pattern is a pattern that generates a steep peak, a pattern of a gentle parabola, etc.) of the line by the cross correlation values for each $\tau$, etc.

In this way, the shape similarity may include various pieces of information, and such information may function as advanced preprocessing data for the estimation model to better extract meaningful features in a later step.

the computation of the cross correlation is performed by at least one of a pre-stored cross correlation scheme, a zero-normalized cross correlation scheme, a normalized cross correlation scheme, and a correlation coefficient scheme. For example, the computation of the cross correlation may be performed based on the following Equations. The above illustrates a cross correlation computation method between the comparison targets in the form of functions, but according to one embodiment, at least one of the comparison targets may be applied to the computation as the set of the coordinate values.

According to one embodiment, the model learning unit 110 may measure the shape similarity between the dataset 111 and the reference pattern 112 by the cross correlation scheme based on Equation 2. The corresponding scheme may be used to quantify the degree of mutual similarity by considering both the shape and the size in which the amplitude changes. For example, even if the similar shape is shown, when the size of the signal value is different, the shape similarity may be measured as a relatively small value.

[Equation 2]

$$r_\tau = \frac{1}{n} \sum_{i=1}^{n-\tau} F_i G_{i+\tau}$$

Here, $F_i$ is a data point in any one of the dataset 111 and the reference pattern 112, and represents a signal value (or a reference signal value) in the corresponding cycle when the cycle number is i. $G_{i+\tau}$ is a data point in the other of the dataset 111 and the reference pattern 112, and denotes a signal value (or a reference signal value) in the corresponding cycle when the cycle number is shifted by $\tau$ to $i + \tau$. In addition, n is a maximum set value (e.g., 45) of the cycle number, and further, $r_\tau$ denotes the correlation values for each $\tau$.

According to one embodiment, the shape similarity may include, as a correlaton value for each $\tau$, the set of the coordinate values ($\tau$, correlation value) measured at each $\tau$ when $\tau$ is changed from a set value $\tau_{min}$ to $\tau_{max}$, or may include a maximum value, an average value, a variance value, a standard deviation value of $r_\tau$, a sum of each value, etc.

According to one embodiment, the model learning unit 110 may measure the shape similarity between the dataset 111 and the reference pattern 112 by the zero-normalized cross correlation scheme based on Equation 3. The corresponding scheme may be used to quantify the degree of mutual similarity by considering the shape with the changing amplitude but compensating for the difference in size.

[Equation 3]

$$r_\tau = \frac{1}{n} \sum_{i=1}^{n-\tau} (F_i - F_{avg})(G_{i+\tau} - G_{avg})$$

Here, $F_{avg}$ and $G_{avg}$ each are the average values of the data points in the dataset 111 and the reference pattern 112 (or the reference pattern 112 and the dataset 111), and denote the results of averaging the signal value (or the reference signal value) in each cycle when the cycle number i changes from 1 to n.

According to one embodiment, the model learning unit 110 may measure the shape similarity between the dataset 111 and the reference pattern 112 by the normalized cross correlation scheme based on Equation 4. The corresponding scheme may be used to quantify the degree of mutual similarity by considering the shape with the changing amplitude but compensating for the difference in scale.

[Equation 4]

$$r_\tau = \frac{1}{n} \frac{\sum_{i=1}^{n-\tau} F_i G_{i+\tau}}{\sqrt{\sum_{i=1}^{n}(F_i - F_{avg})^2 \sum_{i=1}^{n}(G_i - G_{avg})^2}}$$

According to one embodiment, the model learning unit 110 may measure the shape similarity between the dataset 111 and the reference pattern 112 by the correlation coefficient scheme based on Equation 5. The corresponding scheme may be used to quantify the degree of mutual similarity by considering the shape with the changing amplitude but compensating for the difference in size and the difference in scale. The shape similarity $r_\tau$ according to Equation 5 may be measured as a value between -1 and +1 by the above compensation.

[Equation 5]

$$r_\tau = \frac{1}{n} \frac{\sum_{i=1}^{n-\tau}(F_i - F_{avg})(G_{i+\tau} - G_{avg})}{\sqrt{\sum_{i=1}^{n}(F_i - F_{avg})^2 \sum_{i=1}^{n}(G_i - G_{avg})^2}}$$

The description of the measurement of the shape similarity described above is only an example and the present disclosure is not limited thereto. According to another embodiment, the shape similarity may be measured by modifying one or more of the above Equations. According to another embodiment, the shape similarity may be measured by a vector-based measurement scheme that expresses the coordinate values of the comparison targets as vectors and calculates the distance or angle between each vector to obtain the similarity. The vector-based measurement scheme may include, for example, a Euclidean distance scheme, a Manhattan distance scheme, a Minkowski distance scheme, a Cosine similarity scheme, etc. According to another embodiment, the shape similarity may be measured by a contour feature-based similarity measurement scheme that calculates the shape similarity by determining how well the comparison targets are merged, a tangent space representation (TSR)-based measurement scheme that obtains the similarity from the distance and angle relationship and area between the comparison targets, etc. In this way, the shape similarity measurement scheme may be modified and implemented in various forms.

For each of the multiple datasets 111, the model learning unit 110 may calculate the shape similarity 113 for each reference pattern by comparing the target curve generated based on each dataset 111 to each of the multiple reference patterns 112.

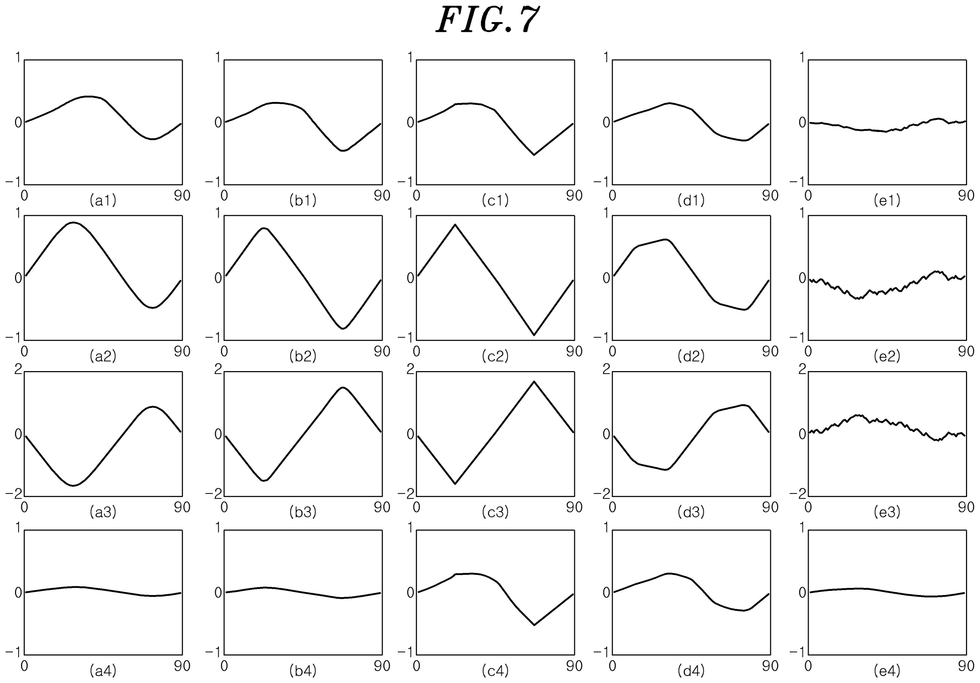

FIG. 7 exemplarily illustrates the shape similarity 113 for each reference pattern according to one embodiment. As illustrated in FIG. 7, the measurement results of each shape similarity may be plotted on the two-dimensional orthogonal coordinate system in which the X-axis is $\tau$ and the Y-axis is the cross correlation value. FIGS. 7A1 to 7E1 illustrate a shape similarity between a first dataset 111a and a first reference pattern 112a to a fifth reference pattern 112e, respectively. FIGS. 7A2 to 7E2 illustrate a shape similarity between a second dataset 111b and the first reference pattern 112a to the fifth reference pattern 112e, respectively. FIGS. 7A3 to 7E3 illustrate a shape similarity between a third dataset 111c and the first reference pattern 112a to the fifth reference pattern 112e, respectively. FIGS. 7A4 to 7E4 illustrate a shape similarity between a fourth dataset 111d and the first reference pattern 112a to the fifth reference pattern 112e, respectively.

According to one embodiment, the shape similarity 113 for each reference pattern may include a set of multiple coordinate values. For example, the shape similarity 113 for each reference pattern between the first dataset 111a and the first reference pattern 112a to the fifth reference pattern 112e, respectively, may include sets of five correlation values for each $\tau$ illustrated in FIGS. 6A1 to 6E1.

Meanwhile, the shape similarity 113 for each reference pattern may be generated as an image type.

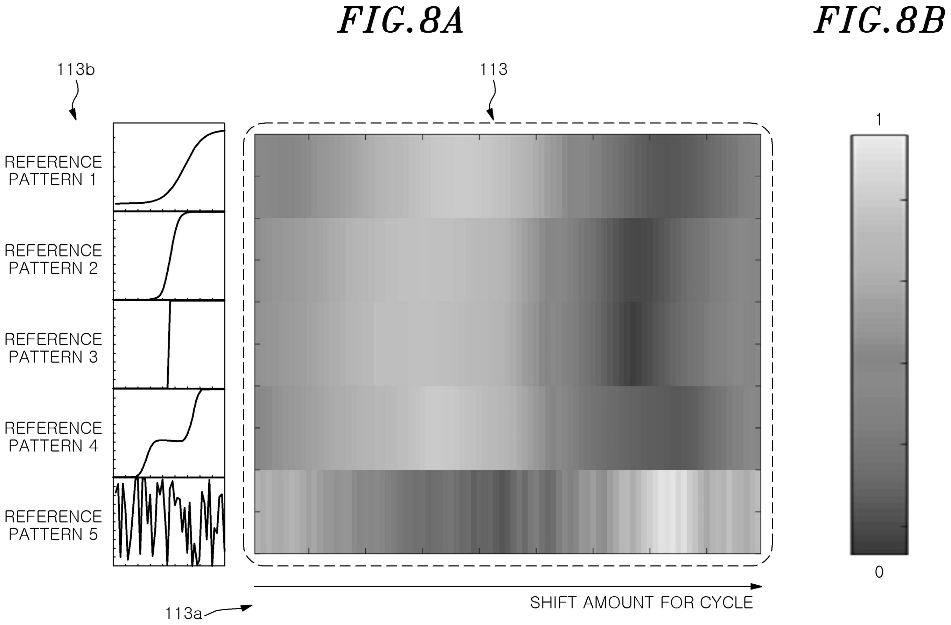

FIG. 8 exemplarily illustrates the shape similarity 113 for each reference pattern generated as an image type according to one embodiment.

Referring to FIG. 8A, the shape similarity 113 for each reference pattern of the image type may be obtained by measuring the shape similarity at each shift amount for each of the multiple reference patterns 112, when shifting any one of the target curve generated based on the dataset 111 and the reference patterns 112 by changing the shift amounts. According to the embodiment, the measured shape similarity may be displayed in an orderly manner for each reference pattern 112 at each shift amount.

According to one embodiment, the shape similarity 113 for each reference pattern of the image type may be a two-dimensional image considering the shift amount and the reference pattern 112. Specifically, in the two-dimensional image, the first axis 113a (e.g., X-axis) denotes $\tau$, which is a shift amount for a cycle, the second axis 113b (e.g., Y-axis) denotes a reference pattern order (e.g., reference pattern number, identifier, etc.), and the coordinate values on the first axis and the second axis may denote the shape similarity measured at each shift amount for the reference pattern 112. For example, as illustrated in FIG. 8, the shape similarity 113 for each reference pattern of the image type may be processed as the set of the coordinate values for the two-dimensional image by aligning the coordinate value sets of FIGS. 7A1 to 7E1 which illustrate the shape similarity between the first dataset 111a and the first reference pattern 112a to the fifth reference pattern 112e, respectively, to the position corresponding to the corresponding reference pattern 112 on the second axis 113b, and aligning each coordinate value to a position corresponding to each shift amount along the first axis 113a.

Referring to FIG. 8B, in the shape similarity 113 for each reference pattern of the image type, the similarity of the target curve for each reference pattern may be divided by color on the image. For example, the range of the shape similarity values is partitioned into multiple sections, and each of the multiple sections is mapped to one of the multiple colors, and the shape similarity 113 for each reference pattern of the image type may include a color value representing a color mapped to the section to which each shape similarity value belongs. The size of the similarity may be represented by color. For example, a larger similarity may be represented in yellow, and a smaller similarity may be represented in blue.

According to another embodiment, the shape similarity 113 for each reference pattern may be generated as a matrix type or a coordinate value set type. Specifically, the shape similarity 113 for each reference pattern may be a multidimensional matrix or the set of the coordinate values in which the values of the shape similarity are aligned for each reference pattern 112 by each shift amount. For example, in the multidimensional matrix, a row represents a shift amount $\tau$ for a cycle, a column represents a reference pattern order, and component values in the row and column may represent shape similarity measured at each shift amount for the corresponding reference pattern 112. Meanwhile, the description of the type of the shape similarity 113 for each reference pattern described above is only an example and the present disclosure is not limited thereto.

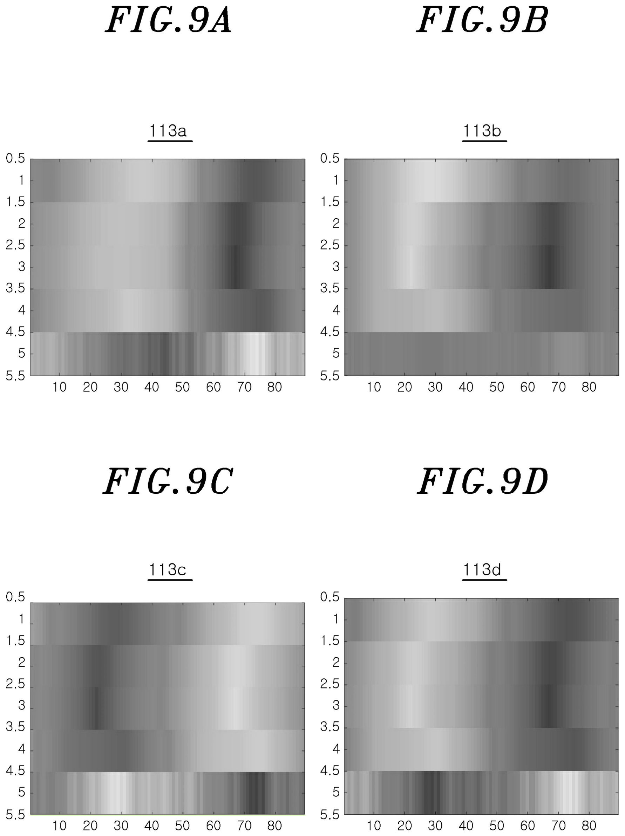

FIG. 9 exemplarily illustrates the shape similarity 113 for each reference pattern of an image type according to one embodiment. FIG. 9A illustrates a first shape similarity 113a for each reference pattern calculated from the first dataset 111a and the first reference pattern 112a to the fifth reference pattern 112e. FIG. 9B illustrates a second shape similarity 113b for each reference pattern calculated from the second dataset 111b and the first reference pattern 112a to the fifth reference pattern 112e. FIG. 9C illustrates a third shape similarity 113c for each reference pattern calculated from the third dataset 111c and the first reference pattern 112a to the fifth reference pattern 112e. FIG. 9D illustrates a fourth shape similarity 113d for each reference pattern calculated from the fourth dataset 111d and the first reference pattern 112a to the fifth reference pattern 112e.

As illustrated in FIG. 9, in the shape similarity 113 for each reference pattern, the time point (e.g., shift amount for the cycle closest to yellow) when one dataset 111 is most similar in shape to each of the multiple reference patterns 112, the section (e.g., range of shift amount close to yellow) in which the shape is similar, the pattern (e.g., pattern in which color changes) in which the degree of shape similarity changes, etc., may appear as colors on the image. Since the shape similarity 113 for each reference pattern is provided as an input to the estimation model in the subsequent process, the estimation model may extract meaningful features for the Ct, the quantitative value, the positive/negative determination result for the target analyte in the sample, or the suitability assessment result of oligonucleotide candidates from the pieces of information contained in the images.

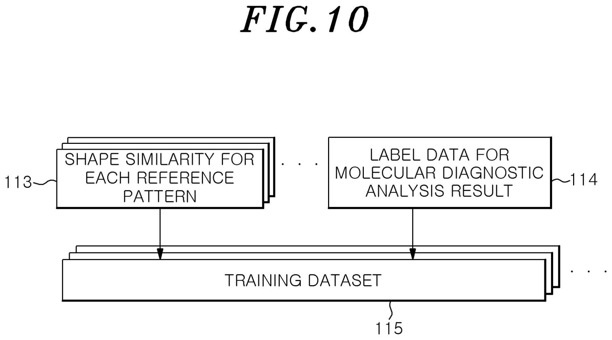

FIG. 10 exemplarily illustrates a conceptual diagram of a process of obtaining multiple training datasets 115 according to one embodiment.

Referring to FIG. 10, the model learning unit 110 may obtain label data 114 for the molecular diagnostic analysis results corresponding to each of the multiple datasets 111 in order to obtain the multiple training datasets 115. According to one embodiment, the label data 114 is label data for the molecular diagnostic analysis results including at least any one selected from the group consisting of the Ct, the quantitative value, the positive/negative determination result for the target analyte in the sample, and the suitability assessment result of oligonucleotide candidates, and may be, for example, data labeled with the shape similarity 113 for each reference pattern as the Ct, the quantitative value, the positive/negative determination result for the target analyte in the sample, or the suitability assessment result of the ligonucleotide candidates. According to one embodiment, the model learning unit 110 may obtain the label data 114 for each molecular diagnostic analysis result when obtaining the multiple datasets 111. According to one embodiment, the model learning unit 110 may obtain the label data 114 for the shape similarity 113 for each of the multiple reference patterns generated by another device and the molecular diagnostic analysis results corresponding to each of the multiple reference patterns from the corresponding device.

Here, the label data 114 for the molecular diagnostic analysis results refers to information labeled as the molecular diagnostic analysis results for the target analyte in the sample in which the amplification reaction, represented by each dataset 111, is performed. According to one embodiment, the label data 114 for the molecular diagnostic analysis results includes at least one of information on the Ct in the target curve generated based on the dataset 111, information on the quantitative value of the target analyte in the corresponding sample, information on the positive/negative determination result for the target analyte in the sample representing the presence or absence of the corresponding target analyte in the corresponding sample, and information on the suitability assessment result of oligonucleotide candidates to be used for the corresponding amplification reaction. According to one embodiment, the Ct in the Ct information included in the label data 114 may be the Ct calculated from each dataset 111 using the pre-stored conventional method (e.g., threshold method, first differentiation method, etc.), or may be the Ct (e.g., the Ct value matching the corresponding quantitative value in the standard curve) corresponding to the quantitative value (e.g., a concentration value set during an experiment) of the target analyte designed in the corresponding amplification reaction. In addition, according to one embodiment, the quantitative value in the quantitative value information included in the label data 114 may be the quantitative value (e.g., the concentration value set during the experiment) of the target analyte designed in the corresponding amplification reaction, or may be the quantitative value (e.g., the concentration value derived from the dataset 111 by applying the absolute quantitation technique based on the standard curve) of the target analyte derived by the quantitation analysis of the result of the corresponding amplification reaction. In addition, according to one embodiment, the positive/negative in the positive/negative determination result information included in the label data 114 may be a result read from each dataset 111 using the pre-stored conventional method (e.g., removing the background signal, calculating the Ct by applying the predetermined signal analysis algorithm to the amplification curve, etc.). In addition, according to one embodiment, the suitability in the suitability assessment result information of the oligonucleotide candidates included in the label data 114 may be a result assessed for each dataset 111 using the pre-stored conventional method (e.g., calculating various experimental values for selecting an oligonucleotide, calculating oligonucleotide performance scores using the experimental values, etc.). The range of the molecular diagnostic analysis results is partitioned into the multiple sections, and each of the multiple sections may be mapped to one of the multiple classes.

According to one embodiment, the Ct information of the label data 114 may include the Ct value in the target curve generated based on the corresponding dataset 111, and may include, for example, a predetermined cycle value input according to a preset unit (e.g., cycle number).

According to another embodiment, the Ct information of the label data 114 may include a class representing a section to which the Ct value in the target curve generated based on the corresponding dataset 111 belongs. Specifically, the range of the Ct is partitioned into the multiple sections, and each of the multiple sections may be mapped to one of the multiple classes. For example, referring to Table 1, the Ct information may include a section to which the corresponding Ct value belongs among the multiple classes and a class mapped thereto, and may be processed from the corresponding Ct value according to one embodiment.

[Table 1]

| Multiple classes | Multiple sections for range of Ct |
|---|---|
| 0 | $T_0$ or more but less than $T_1$ |
| 1 | $T_1$ or more but less than $T_2$ |
| ... | ... |
| N | $T_N$ or more |

Here, N denotes an integer representing the number (e.g., 30, 50, etc.) of classes that can be set by a user, and $T_0$ to $T_N$ each denotes a setting value (e.g., 1, 2, etc.) for the Ct of each section that can be set by the user. According to one embodiment, each of N and $T_0$ to $T_N$ may be set to a different value in consideration of the type of the target analyte, and as each section is subdivided into multiple classes, a more detailed Ct value section may be applied in the Ct estimation process. According to one embodiment, in the case of the amplification reaction performed targeting two or more types of target analytes in the sample in the single channel, the Ct information of the label data 114 may include the Ct information for one of the two or more types of target analytes, include two or more pieces of the Ct information for the two or more types of target analytes, or include Ct set information processed from the two or more pieces of the Ct information. In this case, each class may be, for example, a class to which each Ct value belongs, a class to which a combination of the corresponding Ct sets belongs, or a class notifying that there are two or more Cts.

According to one embodiment, the quantitative value information of the label data 114 may include the quantitative value for the initial concentration of the target analyte included in the corresponding sample, and may include, for example, a predetermined absolute quantitative value input according to the preset unit (e.g., ng).

According to another embodiment, the quantitative value information of the label data 114 may include the class representing the section to which the corresponding the quantitative value belongs. Specifically, the range of the quantitative value is partitioned into the multiple sections, and each of the multiple sections may be mapped to one of the multiple classes. For example, referring to Table 2, the quantitative value information may include the class mapped to the section to which the corresponding absolute quantitative value belongs among the multiple classes, and may be processed from the quantitative value corresponding to the corresponding dataset 111 according to one embodiment.

[Table 2]

| Multiple classes | Multiple sections for range of quantitative value |
|---|---|
| 0 | 0 or more but less than $C_0$ |
| 1 | $C_0$ or more but less than $C_1$ |

(continued)

| Multiple classes | Multiple sections for range of quantitative value |
|---|---|
| ... | ... |
| N | $C_{N-1}$ or more but less than $C_N$ |

Here, each of $C_0$ to $C_N$ represents a setting value (e.g., $10^{-3}$, $10^{-2}$, etc.) for the range of each section that may be set by the user. According to one embodiment, each of N and C0 to CN may be set to a different value in consideration of the type of the target analyte, and as each section is subdivided to be divided into multiple classes, a more detailed quantitative value section may be applied in the quantitative value estimation process. According to one embodiment, the positive/negative determination result information of the label data 114 may include the class representing the presence or absence of the corresponding target analyte in the corresponding sample. For example, the negative representing the absence of the target analyte may be mapped to a first class (e.g., class 0), and the positive representing the presence of the target analyte may be mapped to a second class (e.g., 1).

According to another embodiment, the positive/negative determination result information of the label data 114 may include a class representing the presence or absence of the corresponding target analyte in the corresponding sample, but may include multiple classes that subdivide the degree of the amount of presence. For example, the negative may be mapped to the first class (e.g., class 0), the strong positive indicating the presence of a relatively high concentration of the target analyte may be mapped to the second class (e.g., 1), and the weak positive indicating the presence of the relatively low concentration of the target analyte may be mapped to a third class (e.g., 1).

According to one embodiment, the suitability assessment result information of the oligonucleotide candidates of the label data 114 may include a class representing whether the oligonucleotide used in the corresponding amplification reaction is suitable as the oligonucleotide candidate to be used in the amplification reaction for detecting the corresponding target analyte. For example, the unsuitability may be mapped to the first class (e.g., class 0), and the suitability may be mapped to the second class (e.g., 1).

According to another embodiment, as the suitability degree described above is generated as a numerical value, the suitability assessment result information of the oligonucleotide candidates of the label data 114 may include a class representing a section to which the corresponding suitability value belongs. In the scheme similar to the foregoing, the range of the suitability value is partitioned into the multiple sections, and each of the multiple sections may be mapped to one of the multiple classes.

The model learning unit 110 may obtain the training dataset 115 including the shape similarity 113 for each reference pattern and the label data 114 for the corresponding the molecular diagnostic analysis results. Here, the shape similarity 113 for each reference pattern may be training input data among the training dataset 115, and the label data 114 for the corresponding the molecular diagnostic analysis results may be training ground truth data.

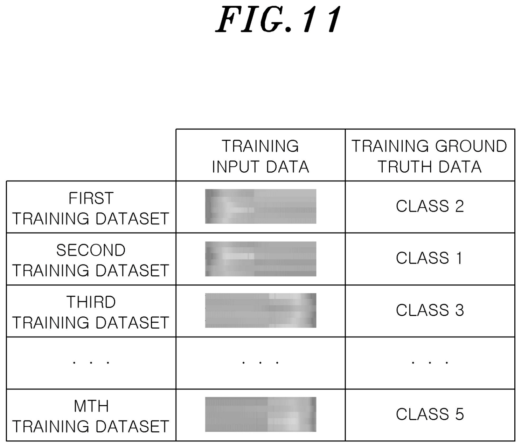

FIG. 11 exemplarily illustrates the multiple training datasets 115 according to one embodiment.

As illustrated in FIG. 11, the model learning unit 110 may generate the multiple training datasets 115 by the scheme that labels the label data 114 for the molecular diagnostic analysis results for the shape similarity 113 for each of the multiple reference patterns obtained from each of the multiple datasets 111 according to the above-described embodiment. For example, an Mth training dataset (M is a natural number) may include an Mth shape similarity for each reference pattern obtained from an Mth dataset as the training input data, a section to which the corresponding Ct value belongs, a section to which the corresponding quantitative value belongs, an Mth class mapped to the corresponding positive and negative, or an Mth class (e.g., 5) mapped to the corresponding suitability as training ground truth data.

According to the embodiment, the training dataset 115 may be obtained by considering the shape of the amplification curve, a distribution of the Ct value, a distribution of the quantitative value, a distribution of the positive and negative, and/or a distribution of the suitability. For example, some datasets may be selectively used among numerous datasets collected in the database so that the size distribution of the Ct value is relatively uniform, the size distribution of the quantitative value is relatively uniform, the distribution of the positive and negative is relatively uniform, the distribution of the suitability and unsuitability or the size distribution of the suitability value is relatively uniform, and the case distribution of various factors affecting the shape of the amplification curve or the amplification reaction is relatively uniform.

According to the embodiment, the processes may be performed sequentially or separately for each dataset 111. For example, the process of sequentially generating the shape similarity 113 for each reference pattern and the training dataset 115 for each dataset 111 may be repeated, and after obtaining all shape similarities 113 for each of the multiple reference patterns for the multiple datasets 111, the multiple training datasets 115 may be obtained at once.

Meanwhile, in the preprocessing process, various conventional preprocessing techniques for preprocessing text or images, etc., that are already known, may be used together. For example, these preprocessing techniques may include a text tokenization technique that divides text values for labeling, a label transformation technique that encodes strings, an image transformation technique that vectorizes images into multidimensional matrices for computation in a deep learning model, and an image processing technique that unifies the size, resolution, color representation scheme, etc. of the images, etc.

Process of training estimation model

The model learning unit 110 may perform a process of training an estimation model using the multiple training datasets 115. Specifically, the model learning unit 110 trains the deep learning model using the training data 115, and when the shape similarity 113 for each reference pattern is provided as a training result, the estimation model trained to estimate the molecular diagnostic analysis results including at least one selected from the group consisting of the Ct in the corresponding target curve, the quantitative value of target analyte in the corresponding sample, the corresponding positive/negative determination result for the target analyte in the sample, and the suitability assessment result of oligonucleotide candidates to be used for the corresponding amplification reaction may be obtained.

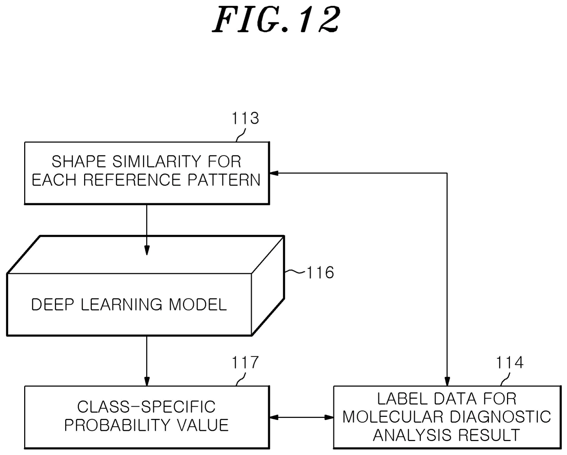

FIG. 12 exemplarily illustrates a conceptual diagram of the process of training the estimation model according to one embodiment. Here, a deep learning model 116 may correspond to the estimation model before the training is completed.

Referring to FIG. 12, the estimation model may be trained to output class-specific probability values 117 from the deep learning model 116 using the multiple training datasets 115. Specifically, in the training process, the estimation model may output the class-specific probability values 117 for the corresponding the molecular diagnostic analysis results based on the shape similarity 113 for each reference pattern that is the training input data. In addition, supervised learning may be performed using a backpropagation scheme so that an error between the output result according to the input and the training ground truth data is minimized.

For example, in the process of training the estimation model, as features are extracted from the shape similarity 113 for each reference pattern input to the model, the class-specific probability values 117 that include probability values (e.g., 93%, 2%, 0.3%, etc.) that the Ct value in the corresponding target curve belongs to each of the multiple sections, probability values that the quantitative value of the target analyte in the corresponding sample belongs to each of the multiple sections, probability values (e.g., 97%, 3%) for each of the positive and negative, or probability values that the suitability values of the oligonucleotide candidates belong to each of the multiple sections may be output. An error may be calculated by comparing the class-specific probability values 117, which are the output results, with the label data 114 (e.g., class number) for the molecular diagnostic analysis results labeled as the ground truth in the shape similarity 113 for each reference pattern, and such an error may be transmitted to the artificial neural network of the deep learning model 116 by the backpropagation scheme to reduce the error. As a result, the parameters included in the artificial neural network, such as weights and bias values, may be updated so that the error may be minimized.

This training process may be performed until the performance of the deep learning model 116 satisfies a predetermined criterion. According to the embodiment, the predetermined criterion may be determined by various schemes, and may be determined by, for example, already known cross validation. In addition, multiple hyperparameters may be used in the training process. The hyperparameters are variables that may vary by the user, and may include, for example, a learning rate, a cost function, the number of learning cycle repetitions, weight initialization, the number of hidden units, a step size (gradient accumulation step) and a batch size during gradient descent learning, etc.

In addition, various optimization methods may be used to prevent an overfitting phenomenon which increases errors for actual data due to excessive training of training data. For example, methods such as increasing training data, regularization, dropout, and utilizing a batch normalization layer may be applied.

According to one embodiment, the shape similarity 113 for each reference pattern generated as an image type in the training process may be used as an input. According to one embodiment, when the shape similarity 113 for each reference pattern of the image type is used, a CNN showing high performance in image recognition may be at least partially borrowed into the deep learning model 116.

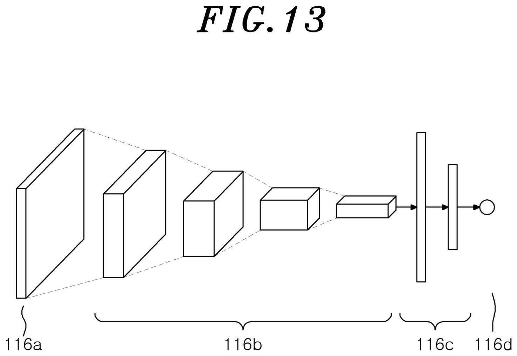

FIG. 13 exemplarily illustrates a schematic structure and operation of a CNN-based deep learning model 116 used for training according to one embodiment. According to one embodiment, the CNN-based deep learning model 116 means a classification model that outputs multiple probability values for each of the multiple classes based on features included in an input image.

Referring to FIG. 13, the CNN-based deep learning model 116 includes a feature extraction layer 116b that extracts features from input data 116a and a classification layer 116c that performs classification on the extracted features and outputs output data 116d as a classification result. Here, the input data 116a corresponds to the shape similarity 113 for each reference pattern of the image type, and the output data 116d corresponds to the class-specific probability value 117.

According to one embodiment, the feature extraction layer 116b may be configured in the form that a convolution layer, a pooling layer, etc., are stacked in multiple layers. Here, the convolution layer may perform a function of extracting features through convolution computation by applying a filter, which is a weight set, to the input data 116a, and may be synthesized with an activation function (e.g., ReLU). In addition, the pooling layer may perform a function such as demension reduction to reduce the size of the image by converting local parts of the input data 116a into a single representative scalar value, and for example, max pooling, average pooling, or min pooling may be used. According to the embodiment, the already known CNN techniques, such as stride or padding, may be applied. In this way, the feature extraction layer 116b may output the features of the image in the form of a feature map while maintaining spatial information of the image by going through a process of repeating the convolution layer that extracts the features of the image through a filter and the pooling layer that strengthens the features and reduces an image dimension.

According to one embodiment, the classification layer 116c may include a flatten layer that changes a data type into a fully connected form, a neural network with a fully connected structure, and an activation function. For example, the extracted features in the form of the feature map are transformed into the fully connected form by the flatten layer and input to a feed forward neural network, and a softmax function may be used as the activation function of the neural network output layer. A vector of a specific dimension output from the neural network may be transformed into a vector with a real value between 0 and 1 and a total sum of 1 by going through the softmax function and output as the class-specific probability value 117.

As the training using the CNN-based deep learning model 116 is performed, the estimation model trained to extract the features appearing in the shape similarity 113 for each reference pattern of the image type and estimate the class to which the molecular diagnostic analysis results belong may be implemented.

In some embodiments, in addition to the CNN described above, various other models capable of image recognition may be used as the deep learning model 116.

In another embodiment, the deep learning model 116 may include a DNN (Deep Neural Network). For example, the deep learning model 116 may be implemented as a DNN having a fully connected neural network architecture. In such a case, shape similarity 113 for each reference pattern of image type may be converted into a one-dimensional array, and then trained using a fully connected multi-layered neural network.

In further embodiment, the deep learning model 116 may include an RNN (Recurrent Neural Network). For example, the deep learning model 116 may be implemented as an RNN having a many-to-many structure that receives multiple data as sequence input and outputs multiple data. In such a case, shape similarity 113 for each reference pattern, which is generated as a coordinate value set of shift amounts and cross-correlation values for cycles, may be trained using the many-to-many RNN structure. Alternatively, the deep learning model 116 may include an LSTM (Long Short-Term Memory) in a manner similar to the embodiments described above. Although RNNs and LSTMs are mainly used for time-series data rather than images, the pixels of adjacent regions within an image are correlated with each other, and thus, image classification may be performed by preprocessing such image data as sequence data.

In further embodiment, the deep learning model 116 may include a ViT (Vision Transformer). The ViT refers to a model that applies a transformer architecture to image classification. For example, the deep learning model 116 may be implemented as a ViT architecture including an embedding layer that receives an image, divides it into multiple patches, and vectorizes the patches, and an encoder of a transformer that outputs context vectors for the respective patches. In this case, shape similarity 113 for each image-type reference pattern may be divided into multiple patches, each of which is flattened and vectorized, and then trained using the encoder.

In further embodiment, the deep learning model 116 may be obtained through pre-training, and an inference model may be implemented by fine-tuning the pre-trained deep learning model 116. For example, a CNN or ViT may be used, and the deep learning model 116 may be pre-trained using a large image dataset related to various graph shapes, and then fine-tuned using training datasets 115 related to nucleic acid amplification reactions to implement the inference model.

Meanwhile, according to one embodiment, information on at least one of a target analyte condition, an environmental condition, and a reaction condition may be further utilized in the process of training the estimation model.

Here, the target analyte condition refers to conditions on the type or concentration of the target analyte targeted by the nucleic acid amplification reaction, and may include, for example, species of living organisms to be detected. In addition, the environmental conditions may broadly refer to surrounding conditions affecting the nucleic acid amplification reaction, and may include types such as a reaction vessel, a plate, a nucleic acid extraction device, and a detection device in which the nucleic acid amplification reaction is performed, etc. In addition, the reaction conditions may broadly refer to conditions for the reaction environment of the nucleic acid amplification reaction or materials added to form the reaction environment, etc. According to one embodiment, the reaction conditions may include at least one of a reaction medium and other conditions (e.g., temperature, pressure, time) used in the nucleic acid amplification reaction. The reaction medium is a material surrounding the reaction environment, and according to one embodiment, may include materials added to form the reaction environment so that one or more of the multiple steps (e.g., denaturation step, annealing step, and extension (or amplification) step) for the nucleic acid amplification reaction may be performed. According to one embodiment, the reaction medium may be one or more materials selected from the group consisting of a pH-related material (e.g., tris buffer), an ionic strength-related material (e.g., ionic material), an enzyme, and an enzyme stabilization-related material (e.g., sugar).

Multiple estimation models according to one embodiment may be prepared by considering at least one of the above-described conditions. For example, the estimation model may be individually prepared for each of the multiple condition groups set in consideration of the type of target analyte (e.g., target product group), the type of detection device, and/or the reaction conditions, and the process of training of each estimation model may be performed using the training datasets 115 tested under each condition group.

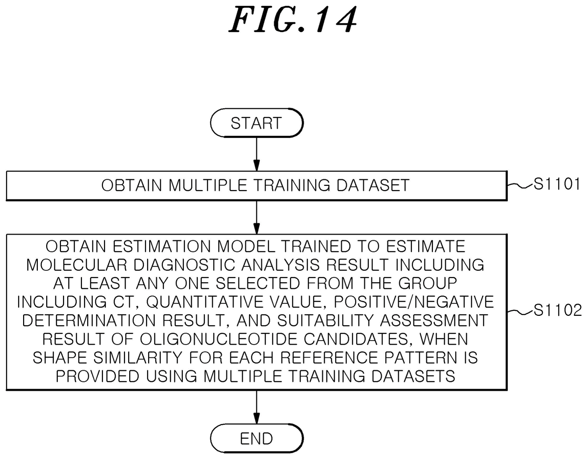

FIG. 14 illustrates an exemplary flowchart for a computer device 1000 according to one embodiment to train the estimation model. According to one embodiment, the steps of FIG. 14 may be implemented by one entity, such as a scheme performed by a server. According to another embodiment, the steps of FIG. 14 may be implemented by multiple entities, such as a scheme in which some of the steps are performed by a user terminal and others performed by a server.

Referring to FIG. 14, in step S1101, the computer device 1000 may obtain the multiple training datasets 115. As described above, each training dataset 115 may include (a) the training input data including the shape similarity 113 for each reference pattern by comparing the target curve generated based on each dataset 111 to each of the multiple pre-established reference patterns 112, and (b) the training ground truth data including the label data 114 for the molecular diagnostic analysis results including at least any one selected from the group consisting of the Ct information in the target curve generated based on each dataset 111, the quantitative value information of the target analyte in the corresponding sample, the positive/negative determination result for the target analyte in the sample, and the suitability assessment result of oligonucleotide candidates to be used for the corresponding amplification reaction.

According to a first embodiment, the training ground truth data of each training dataset 115 may include label data for the Ct information in the target curve generated based on each dataset 111.

According to a second embodiment, the training ground truth data of each training dataset 115 may include label data for the quantitative value information of the target analyte in each sample.

According to a third embodiment, the training ground truth data of each training dataset 115 may include label data for the positive/negative determination result information about the presence or absence of the target analyte in each sample.

According to a fourth embodiment, the training ground truth data of each training dataset 115 may include label data for suitability assessment result information of oligonucleotide candidates to be used for each amplification reaction.

According to a fifth embodiment, the training ground truth data of each training dataset 115 may include label data for at least some combination of the Ct information, the quantitative value information, the positive/negative determination result for the target analyte in the sample, and the suitability assessment result of oligonucleotide candidates.

According to a sixth embodiment, the multiple training datasets 115 may include training datasets for the Ct, each of which includes the shape similarity 113 for each reference pattern and the label data for the Ct information, training datasets for the quantitative value, each of which includes the shape similarity 113 for each reference pattern and the label data for the quantitative value information, training datasets for the positive/negative determination result for the target analyte in the sample, each of which includes the shape similarity 113 for each reference pattern and the label data for the positive/negative determination result for the target analyte in the sample, and training datasets for the suitability assessment result of oligonucleotide candidates, each of which includes the shape similarity 113 for each reference pattern and the label data for suitability assessment result.

In step S1102, the computer device 1000 may obtain the estimation model trained to estimate the molecular diagnostic analysis results including at least any one selected from the group consisting of the Ct, the quantitative value, the positive/negative determination result for the target analyte in the sample, and the suitability assessment result of oligonucleotide candidates by using the multiple training datasets 115, when the shape similarity 113 for each reference pattern is provided. According to the first embodiment, the computer device 1000 may train the estimation model to estimate the Ct. The computer device 1000 may input the shape similarity 113 for each reference pattern to the estimation model, and train the estimation model to minimize errors by comparing the class-specific probability values for the Ct output from the estimation model with the training ground truth data.

According to the second embodiment, the computer device 1000 may train the estimation model to estimate the quantitative value. The computer device 1000 may input the shape similarity 113 for each reference pattern to the estimation model and train the estimation model to minimize errors by comparing the class-specific probability value of the quantitative value output from the estimation model with the training ground truth data.

According to the third embodiment, the computer device 1000 may train the estimation model to estimate the positive/negative determination result for the target analyte in the sample. The computer device 1000 may input the shape similarity 113 for each reference pattern to the estimation model and train the estimation model to minimize errors by comparing the class-specific probability values of the positive/negative determination result for the target analyte in the sample output from the estimation model with the training ground truth data.

According to the fourth embodiment, the computer device 1000 may train the estimation model to estimate the suitability assessment result of oligonucleotide candidates. The computer device 1000 may input the shape similarity 113 for each reference pattern to the estimation model, and compare the class-specific probability value of the suitability value of oligonucleotide candidates output from the estimation model with the training ground truth data to train the estimation model so that the errors are minimized.

According to the fifth embodiment, the computer device 1000 may train the estimation model to estimate at least some of the Ct, the quantitative value, the positive/negative determination result for the target analyte in the sample, and the suitability assessment result of oligonucleotide candidates together. The computer device 1000 may input the shape similarity 113 for each reference pattern to the estimation model, and compare the class-specific probability value of the combination of at least some of the Ct information, the quantitative value information, the positive/negative determination result for the target analyte in the sample, and suitability assessment result of oligonucleotide candidates output from the estimation model with the training ground truth data to train the estimation model so that the errors are minimized.

According to the sixth embodiment, the computer device 1000 may train the first estimation model to the fourth estimation model to estimate the Ct, the quantitative value, the positive/negative determination result for the target analyte in the sample, and the suitability assessment result of oligonucleotide candidates, respectively, and obtain the estimation model including the first estimation model to the fourth estimation model as the training results for each of the first estimation model to the fourth estimation model.

The computer device 1000 may store and manage the estimation model, and provide the estimation model. For example, the computer device 1000 may be implemented to store and manage the estimation model trained by the server, and when the user terminal requests the estimation model, the server may provide the estimation model to the user terminal.

Preprocessing process for estimating the molecular diagnostic analysis results

The estimation unit 120 may prepare the estimation of the molecular diagnostic analysis results using the estimation model through the preprocessing process for estimating the molecular diagnostic analysis results. According to one embodiment, the estimation unit 120 may perform the preprocessing process for estimating the molecular diagnostic analysis results when a request for estimating the molecular diagnostic analysis results for a predetermined dataset is received.

The preprocessing process for estimating the molecular diagnostic analysis results according to one embodiment includes a process of preprocessing a dataset for estimating the molecular diagnostic analysis results as the input data to be provided to the estimation model. The preprocessing process may be performed by a scheme substantially similar to the process of preprocessing the dataset for training as the input data to be provided to the estimation model, as described above, and redundant contents thereof will be omitted.

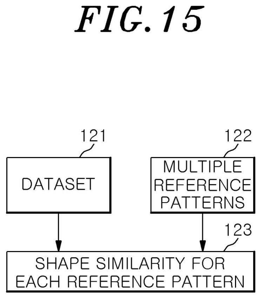

FIG. 15 exemplarily illustrates a conceptual diagram for the process of preprocessing the dataset for estimating the molecular diagnostic analysis results according to one embodiment as the input data to be provided to the estimation model. FIG. 15 may be understood with reference to the embodiments illustrated in FIGS. 3 to 9.

Referring to FIG. 15, the estimation unit 120 may obtain a dataset 121 and multiple reference patterns 122 for estimating the molecular diagnostic analysis results, respectively. According to one embodiment, the estimation unit 120 may obtain the dataset 121 and/or the multiple reference patterns 112 from the memory 100 or the storage device by the processor 300 or from another external device through the communication unit 200.

Here, the dataset 121 is a target for estimating the molecular diagnostic analysis results, and represents the result of the amplification reaction for the target analyte in the sample. The embodiment of the dataset 121 may include at least some of the embodiments described above for the dataset 111 for training. For example, the dataset 121 may include signal values in each of the multiple cycles obtained as a result of the amplification reaction for an unknown sample collected from a test subject, or a first derivative result for a curve connecting the signal values in each of the multiple cycles. In addition, the graphs illustrated in FIG. 4 may be interpreted as exemplifying the target curve generated based on the dataset 121 for estimating the molecular diagnostic analysis results, such as the Ct, the quantitative value, or the positive/negative determination result for the target analyte in the sample. For another example, the dataset 121 may include, as a result of the amplification reaction on the sample in which the oligonucleotide designed as the oligonucleotide candidate for detecting the target analyte and the corresponding target analyte are accommodated together, the signal values or their first derivative results for each of the multiple cycles during the oligonucleotide design process. In addition, the graphs illustrated in FIG. 4 may be interpreted as exemplifying the target curve generated based on the dataset 121 that intends to estimate the molecular diagnostic analysis results such as whether the oligonucleotide used in the sample is suitable as the oligonucleotide candidates to be used in the molecular diagnostic reagent for the detection of the corresponding target analyte or the level of suitability.

The multiple reference patterns 122 correspond to the multiple reference patterns 112 described above, and for example, several reference patterns including the reference patterns illustrated in FIG. 5 may be used together in the preprocessing process for training and the preprocessing process for estimation.

The estimation unit 120 may calculate a shape similarity 123 for each reference pattern by comparing the target curve generated based on the dataset 121 to each of the multiple reference patterns 122. Similarly, the method for calculating the shape similarity 123 for each reference pattern corresponds to the method for calculating the shape similarity 113 for each reference pattern, and the embodiment of the shape similarity 123 for each reference pattern may include the embodiments described above for the shape similarity 113 for each reference pattern. For example, the shape similarity 123 for each reference pattern may be obtained by measuring the shape similarity measured at each shift amount for the cycles with respect to each of the multiple reference patterns 122, as illustrated in FIG. 7, and may be generated as the image type in which the similarity is divided by color, as illustrated in FIGS. 8 and 9.

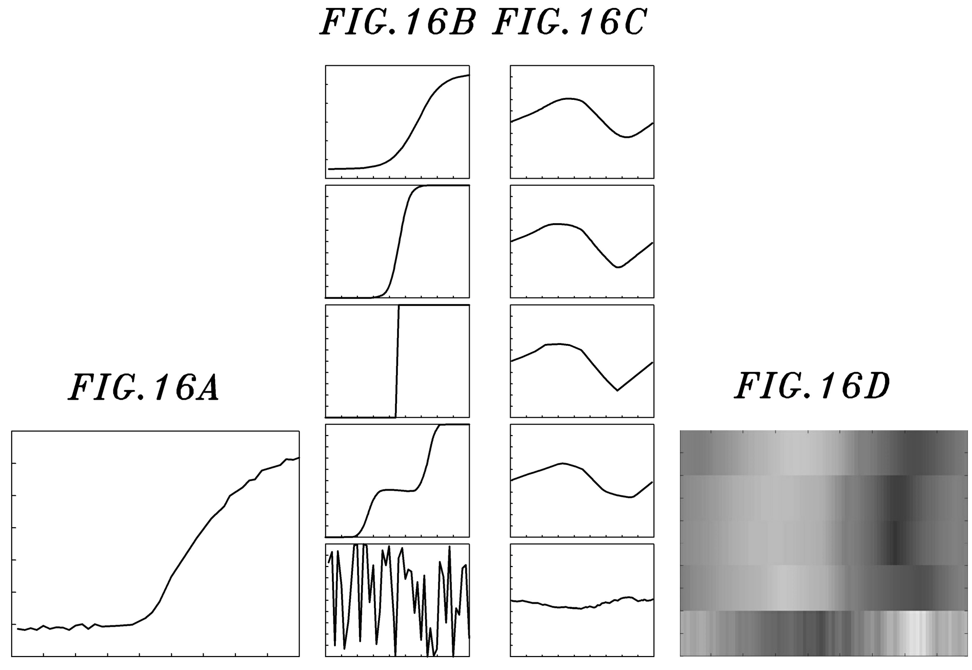

FIG. 16 exemplarily illustrates the dataset 121, the multiple reference patterns 122, and the shape similarity 123 for each reference pattern for estimating the molecular diagnostic analysis results according to one embodiment. FIG. 16A illustrates the dataset 121 for estimating the molecular diagnostic analysis results, and illustrates a case where the amplification curve is relatively close to a normal positive case, exhibiting a shape where the amplification curve is relatively positioned in the middle with the cycle number at which the amplitude reaches the threshold is approximately 25. FIG. 16B illustrates the multiple reference patterns 122. FIG. 16C illustrates the shape similarity measured for each of the multiple reference patterns 122. FIG. 16D illustrates the shape similarity 123 for each reference pattern generated as the image type.

Process of estimating molecular diagnostic analysis results using estimation model

When the preprocessing process for estimating the molecular diagnostic analysis results is performed, the estimation unit 120 may perform a process of estimating the molecular diagnostic analysis results including at least any one selected from the group consisting of the Ct in the corresponding target curve, the quantitative value of the target analyte in the corresponding sample, the positive/negative determination result for the target analyte in the sample, and the suitability assessment result of oligonucleotide candidates to be used for the corresponding amplification reaction using the estimation model.

The process of estimating the molecular diagnostic analysis results according to one embodiment may be performed by a scheme similar to the process of the model obtaining output results according to the input during the process of training the estimation model described above, and similarly, redundant contents thereof will be omitted.

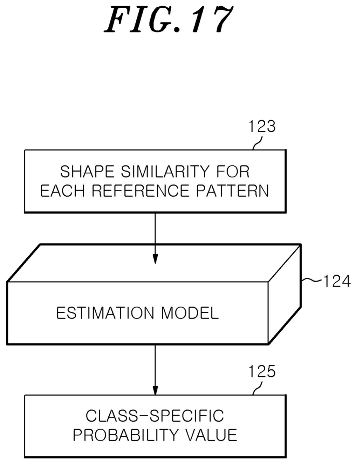

FIG. 17 exemplarily illustrates a conceptual diagram of the estimation process using the estimation model according to one embodiment. Here, an estimation model 124 may correspond to the deep learning model 116 whose training has been completed.

Referring to FIG. 17, the estimation unit 120 may obtain the molecular diagnostic analysis results from the estimation model 124 by providing the shape similarity 123 for each reference pattern to the estimation model 124. Here, obtaining the molecular diagnostic analysis results refers to obtaining the estimation result for the molecular diagnostic analysis results. As described above, the estimation model 124 is trained to estimate the corresponding molecular diagnostic analysis results when receiving the shape similarity 123 for each reference pattern, and therefore, may extract features from the shape simillarity 123 for each reference pattern, which serves as the input data, by using the weights, the bias values of the neural network obtained through the training to output the estimation result for the Ct in the corresponding target curve, the quantitative value of the target analyte in the corresponding sample, the positive/negative determination result for the target analyte in the sample, and/or the suitability assessment result of oligonucleotide candidates to be used for the corresponding amplification reaction.

According to one embodiment, the estimation result for the Ct may include a class representing a section to which the corresponding Ct value belongs. According to another embodiment, the estimation result for the Ct may include the probability values for each of the multiple classes. For example, referring to Table 3, the estimation model 124 may output a class-specific probability value 125 representing the probability values for each of the multiple classes as the estimation result for the Ct, or may output one or more classes having the largest probability value among the multiple classes or satisfying a preset condition (e.g., the threshold for the probability value) and the probability values of the one or more classes.

[Table 3]

| Multiple classes | Multiple sections for range of Ct | Probability value |
| --- | --- | --- |
| 0 | $T_0$ or more but less than $T_1$ | 0.03 |
| 1 | $T_1$ or more but less than $T_2$ | 0.75 |
| ... | ... | ... |
| N | $T_N$ or more | 0.01 |

According to one embodiment, the estimation result for the quantitative value may include the class representing the section to which the quantitative value of the corresponding target analyte belongs. According to another embodiment, the estimation result for the quantitative value may include the probability values for each of the multiple classes. For example, referring to Table 4, the quantitative value estimation model 124 may output the class-specific probability value 125 representing the probability values for each of the multiple classes as the estimation result for the quantitative value, or may output one or more classes having the largest probability value among the multiple classes or satisfying the preset condition (e.g., the threshold for the probability value) and the probability values of the one or more classes.

[Table 4]

| Multiple classes | Multiple sections for range of quantitative value | Probability value |
| --- | --- | --- |
| 0 | 0 or more but less than $C_0$ | 0.02 |
| 1 | $C_0$ or more but less than $C_1$ | 0.83 |
| ... | ... | ... |
| N | $C_{N-1}$ or more but less than $C_N$ | 0.01 |

According to one embodiment, the estimation result for the positive/negative determination result for the target analyte in the sample may include a class representing whether the determination result is positive or negative. According to another embodiment, the estimation result for the positive/negative determination result for the target analyte in the sample may include the probability values for each of the multiple classes. According to one embodiment, the estimation result for the suitability assessment result of oligonucleotide candidates may include a class representing whether the oligonucleotide is suitable or unsuitable. For another embodiment, the estimation result for the suitability assessment may include the probability values for each of the multiple classes.

According to one embodiment, the estimation model 124 may receive the shape similarity 123 for each reference pattern generated as the image type as the input. For example, the estimation model 124 may receive the shape similarity 123 for each reference pattern processed by the image so that the similarity is divided by color, as illustrated in FIG. 16D, and may perform feature extraction and classification from the image through the feature extraction layer 116b and the classification layer 116c, as illustrated in FIG. 13, and output the class-specific probability value 125.

Accordingly, the Ct in the corresponding target curve, the quantitative value of the target analyte in the corresponding sample, the positive/negative determination result for the target analyte in the sample, and/or the suitability assessment result of oligonucleotide candidates to be used for the corresponding amplification reaction may be easily estimated from the information for the molecular diagnostic analysis results included in the shape similarity 123 for each reference pattern. In addition, the shape similarity 123 for each reference pattern includes not only information on whether the shape of the target curve is similar to the ideal growth curve, but also various information described above, so even when the target curve does not follow the general pattern of being divided into the baseline region, the exponential region, and the plateau region due to various environmental causes, the molecular diagnostic analysis results may be easily estimated through a shape similarity with other reference patterns.

According to one embodiment, the estimation unit 120 may compute by a predetermined scheme using the probability values for each of the multiple classes, and obtain a corrected estimation result from the estimation result for the molecular diagnostic analysis results using the computation result. For example, in the case of the Ct estimation, the estimation unit 120 may calculate a result of summing the product of the representative value (e.g., minimum value, median value, maximum value) of each section and the corresponding probability values for each section, and determine that the value according to the summed result is the Ct value, or obtain the corrected estimation result in which the section to which the corresponding value belongs is determined to be the section to which the Ct value belongs.

Meanwhile, in one embodiment where the amplification reaction targets two or more types of target analytes as described above, the estimation unit 120 may provide the estimation result for two or more molecular diagnostic analysis results. For example, when two types of target analytes is present at different concentrations in the sample, the target curve generated based on the dataset 121 may appear as the shape in which two different sigmoid curves, etc., overlap each other. The estimation unit 120 inputs the shape similarity 123 for each reference pattern preprocessed from the dataset 121 to the estimation model 124, and may provide a class corresponding to two Ct values or a set of the corresponding Ct values, a class corresponding to two quantitative values or a set of the corresponding quantitative values, or a class corresponding to two positive/negative determination results or a set of the corresponding positive/negative determination results as the estimation result. Alternatively, when the estimation unit 120 outputs a class notifying that there are two or more Ct, the quantitative value, or the positive/negative determination result for the target analyte in the sample in the estimation model 124, the estimation unit 120 may perform a process of extracting signals dependent on each target analyte from the corresponding dataset 121, and provide each of the two or more preprocessed shape similarities 123 for each reference pattern, from the two or more extracted signals, respectively, to the estimation model 124, thereby providing the estimation result that includes the Ct value, the quantitative value, the positive/negative determination result for the target analyte in the sample, or the suitability assessment result of oligonucleotide candidates for each target analyte or the corresponding classes.

Meanwhile, according to one embodiment, the estimation unit 120 may obtain the estimation result for the molecular diagnostic analysis results from the estimation model 124 by further utilizing the information on at least one of the target analyte conditions, the environmental conditions, and the reaction conditions used in the nucleic acid amplification reaction. For example, when the estimation unit 120 obtains the dataset 121, the estimation unit 120 may obtain the information on the target analyte condition, the environmental condition, and the reaction condition under which the corrensponding amplification reaction was performed, and input the shape similarity 123 for each reference pattern preprocessed from the corresponding dataset 121 to the estimation model 124 corresponding to the condition group that matches the pieces of corresponding condition information among the multiple estimation models 124 provided, thereby obtaining the Ct, the quantitative value, the positive/negative determination result for the target analyte in the sample, or the suitability assessment result of the oligonucleotide candidates.

The estimation unit 120 may provide the estimation result described above by various schemes. According to one embodiment, the estimation unit 120 may output a result screen, as the estimation result, including information on one or more sections that satisfy the preset conditions and the corresponding probability value. For example, conditions such as having a probability value greater than or equal to a predetermined threshold or corresponding to a top specific number in order of the largest value may be applied to these conditions. According to another embodiment, the estimation unit 120 may output the result screen, as the estimation result, including information on multiple sections as the estimation result (e.g., ranges of the corresponding Ct value) and the class-specific probability value 125 mapped to each section. According to the embodiment, the result screen may be displayed in order of probability values from largest to smallest, or one or more sections satisfying the predetermined condition and their probability values may be highlighted. According to another embodiment, the estimation unit 120 may also output the result screen including the estimation result and/or the corrected estimation result.

The result screen may be displayed in the form of a table, graph, or image, etc., according to the implementation aspect, and the type, scale, etc., of the table or the graph may be different.

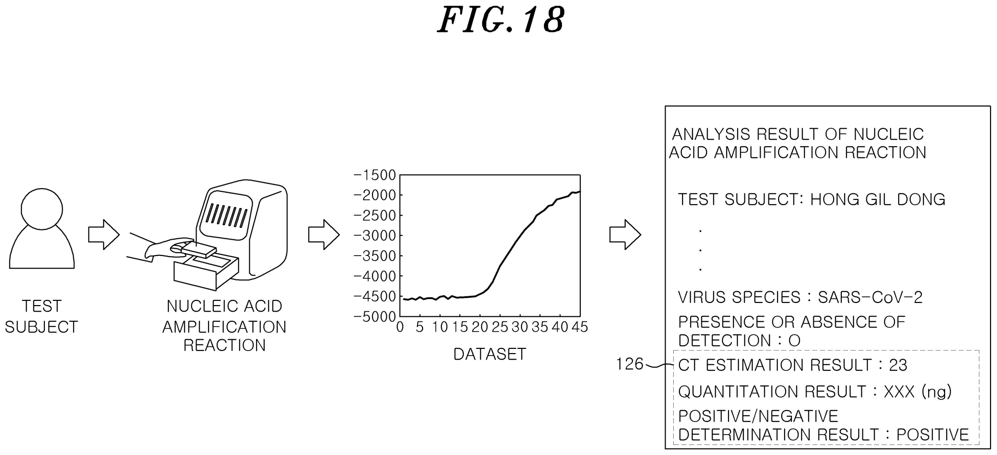

FIG. 18 exemplarily illustrates a process of providing an estimation result for the molecular diagnostic analysis results according to one embodiment. As illustrated in FIG. 18, the nucleic acid extraction and the nucleic acid amplification reaction may be performed using the sample collected from the test subject, and the dataset 121 representing the result of the nucleic acid amplification reaction may be obtained. The computer device 1000 may calculate the shape similarity 123 for each reference pattern by comparing the target curve generated based on the dataset 121 to each of the multiple reference patterns 122, and provide the shape similarity 123 for each reference pattern to the pre-trained estimation model 124, thereby obtaining the estimation result for the molecular diagnostic analysis results from the estimation model 124. The computer device 1000 may provide the analysis results of the nucleic acid amplification reaction including the estimation result, and may output the analysis results including, for example, test subject information, target analyte information (e.g., virus species), whether the target analyte is detected, and an estimation result 126 for the Ct, the quantitative value, and the positive/negative determination result for the target analyte in the sample. In FIG. 18, an example is illustrated in which the Ct value and the quantitative value are displayed as values as the estimation result 126, but according to the embodiment, the section to which the Ct value or the quantitative value belongs may be displayed.

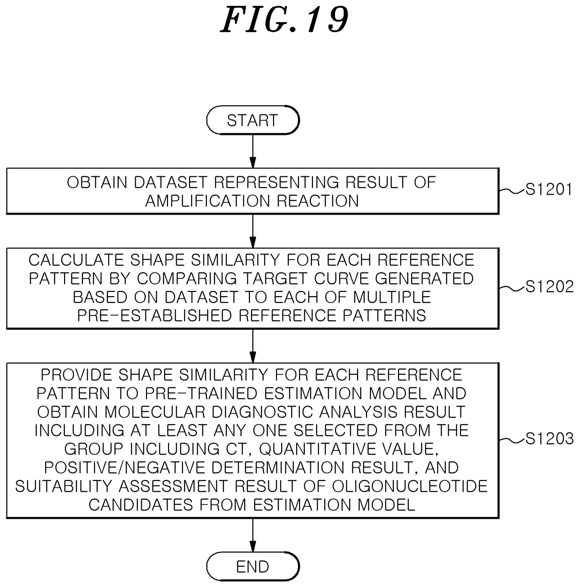

FIG. 19 illustrates an exemplary flowchart for estimating the molecular diagnostic analysis results by the computer device 1000 according to one embodiment. According to one embodiment, the steps of FIG. 19 may be implemented by one entity, such as the scheme performed by the user terminal. According to another embodiment, the steps of FIG. 19 may be implemented by multiple entities, such as a scheme in which some of the steps are performed by a user terminal and others performed by a server.

Referring to FIG. 19, in step S1201, the computer device 1000 may obtain the dataset 121 representing the result of the amplification reaction. According to one embodiment, the amplification reaction may be based on the real-time amplification. According to one embodiment, the dataset 121 may include signal values in each of the multiple cycles obtained as the result of the amplification reaction or an nth derivative result for a curve connecting the signal values in each of the multiple cycles.

In step S1202, the computer device 1000 may calculate the shape similarity 123 for each reference pattern by comparing the target curve generated based on the dataset 121 to each of the multiple pre-established reference patterns 122. According to one embodiment, the shape similarity 123 for each reference pattern may be calculated by computing the cross correlation between the target curve and each of the multiple reference patterns. According to one embodiment, the shape similarity 123 for each reference pattern may be generated as the image type.

In step S1203, the computer device 1000 provides the shape similarity 123 for each reference pattern to the pre-trained estimation model 124, so as to obtain the molecular diagnostic analysis results including at least any one selected from the group consisting of the Ct in a corresponding target curve, the quantitative value of the target analyte in the corresponding sample, the positive/negative determination result for the target analyte in the sample, and the suitability assessment result of oligonucleotide candidates to be used for the amplification reaction from the estimation model 124. According to one embodiment, the estimation model 124 may receive the shape similarity 123 for each reference pattern generated as the image type as the input. According to one embodiment, when the estimation model 124 receives the shape similarity 123 for each reference pattern, the estimation model 124 may output the probability values for each of the multiple classes mapped to each of the multiple sections in which the range of the Ct, the quantitative value, the positive/negative determination result for the target analyte in the sample, or the suitability assessment result of the oligonucleotide candidates is partitioned.

According to the first embodiment, the computer device 1000 may input the shape similarity 123 for each reference pattern to the estimation model 124 trained to estimate the Ct, and output the class-specific probability values for the Ct in the corresponding target curve from the estimation model 124.

According to the second embodiment, the computer device 1000 may input the shape similarity 123 for each reference pattern to the estimation model 124 trained to estimate the quantitative value, and output the class-specific probability values for the quantitative value of the target analyte in the corresponding sample from the estimation model 124.

According to the third embodiment, the computer device 1000 may input the shape similarity 123 for each reference pattern to the estimation model 124 trained to estimate the positive/negative determination result for the target analyte in the sample, and output the class-specific probability value for the positive/negative determination result regarding the presence or absence of the target analyte in the corresponding sample from the estimation model 124.

According to the fourth embodiment, the computer device 1000 may input the shape similarity 123 for each reference pattern to the estimation model 124 trained to estimate the suitability assessment result of oligonucleotide candidates, and output the class-specific probability value for the suitability assessment result of oligonucleotide candidates to be used for the amplification reaction from the estimation model 124.

According to the fifth embodiment, the computer device 1000 may input the shape similarity 123 for each reference pattern to the estimation model 124 trained to estimate at least some of the Ct, the quantitative value, the positive/negative determination result for the target analyte in the sample, and the suitability assessment result of oligonucleotide candidates, and output the class-specific probability values for the combination of at least some of the Ct, the quantitative value, the positive/negative determination result for the target analyte in the sample, and the suitability assessment result of oligonucleotide candidates or the class-specific probability values for each of at least some of the Ct, the quantitative value, the positive/negative determination result for the target analyte in the sample, and the suitability assessment result of oligonucleotide candidates from the estimation model 124.

According to the sixth embodiment, the computer device 1000 may input the shape similarity 123 for each reference pattern to each of the first to fourth estimation models trained to estimate the Ct, the quantitative value, the positive/negative determination result for the target analyte in the sample, and the suitability assessment result of oligonucleotide candidates, and output the class-specific probability values for each of the Ct, the quantitative value, the positive/negative determination result for the target analyte in the sample, and the suitability assessment result of oligonucleotide candidates from each of the first to fourth estimation models.

As described above, the molecular diagnostic analysis results in the corresponding target curve may be estimated from the dataset representing the result of the amplification reaction using the method for estimating the molecular diagnostic analysis results described in the present specification. This technical feature may be used independently, without being combined with the learning method of the estimation model, according to the embodiment.

Embodiments for obtaining molecular diagnostic analysis results

The above has been described focusing on embodiments for estimating the molecular diagnostic analysis results using the estimation model. However, the present disclosure is not limited thereto, and based on the technical idea described above, the computer device 1000 may perform the process for obtaining the molecular diagnostic analysis results.

According to another embodiment of the present disclosure, the estimation unit 120 may be implemented to obtain the molecular diagnostic analysis results regarding the target analyte in the sample by using the shape similarity 123 for each reference pattern.

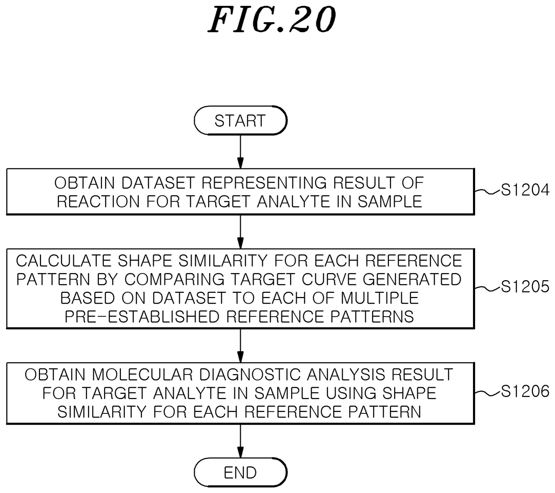

FIG. 20 illustrates an exemplary flowchart for obtaining the molecular diagnostic analysis results by the computer device 1000 according to one embodiment. FIG. 20 may be understood with reference to the embodiments described above.

Referring to FIG. 20, in step S1204, the computer device 1000 may obtain the dataset 121 representing the result of the amplification reaction regarding the target analyte in the sample.

In step S1205, the computer device 1000 may calculate the shape similarity 123 for each reference pattern by comparing the target curve generated based on the dataset 121 to each of the multiple pre-established reference patterns 122.

In step S1206, the computer device 1000 may obtain the molecular diagnostic analysis results for the target analyte in the corresponding sample using the shape similarity 123 for each reference pattern. For example, the shape similarity 123 for each reference pattern may be directly or indirectly used in a process of calculating the molecular diagnostic analysis results that include the positive/negative determination result for the presence or absence of the target analyte in the sample, the Ct value, the absolute quantitative value of the target analyte, the suitability assessment result of oligonucleotide candidates to be used for the corresponding amplification reaction, etc. For example, the computer device 1000 may control the shape similarity 123 for each reference pattern to be used for the calculation of the molecular diagnostic analysis results by transmitting the shape similarity 123 for each reference pattern to the terminal for molecular diagnostic analysis (not illustrated) or applying the shape similarity 123 for each reference pattern to another algorithm (e.g., Equation) for the calculation of the pre-stored molecular diagnostic analysis results. According to another embodiment of the present disclosure, the computer device 1000 may perform a method for calculating a shape similarity for molecular diagnostic analysis. In the method for calculating a shape similarity for molecular diagnostic analysis, in step S1204, the computer device 1000 may obtain the dataset 121 representing the result of the amplification reaction for the target analyte in the sample. In step S1205, the computer device 1000 may calculate the shape similarity 123 for each reference pattern by comparing the target curve generated based on the dataset 121 to each of the multiple pre-established reference patterns 122. In this embodiment, step S1206 may be omitted. For example, the computer device 1000 may output the shape similarity 123 for each reference pattern, store the shape similarity 123 for each reference pattern in the memory 100, or provide the shape similarity 123 for each reference pattern to the user terminal associated with the molecular diagnostic analysis. For example, the computer device 1000 may generate the shape similarity 123 for each reference pattern as the preprocessing data that may be used as the input data in the artificial intelligence-based model, and provide the preprocessing data to the user terminal (e.g., the terminal of the user that requests the preprocessing data) that uses the artificial intelligence-based model.

Meanwhile, it will be understood by those skilled in the art that various exemplary logic blocks, modules, processors, means, circuits, and algorithm steps described in connection with the embodiments disclosed herein may be implemented in electronic hardware, in various forms of programs or design code (collectively referred to herein, for convenience, as software), or in a combination of both hardware and software. To clearly describe the interoperability between hardware and software, various exemplary components, blocks, modules, circuits, and steps have been generally described above in terms of their functionality. Whether such functionality is implemented as hardware or software depends on design constraints imposed on the specific application and overall system. Those skilled in the art will recognize that the described functionality may be implemented in various ways for each particular application; however, such implementation decisions should not be interpreted as departing from the scope of the present disclosure.

Various embodiments described herein may be implemented as methods, devices, or articles of manufacture using standard programming and/or engineering techniques. As used herein, the term "article of manufacture" includes a computer program accessible from any computer-readable storage device, carrier, or media. For example, the computer-readable storage media may include, but are not limited to, magnetic storage devices (e.g., hard disks, floppy disks, magnetic tape), optical disks (e.g., CDs, DVDs), smart cards, and flash memory devices (e.g., EEPROMs, cards, sticks, key drives). Furthermore, the various storage media presented herein may include one or more devices and/or other machine-readable media for storing information.

It should be understood that the specific order or hierarchy of steps in the processes described herein is merely an example of possible approaches. Based on design preferences, the specific order or hierarchy of the steps in the processes may be rearranged while remaining within the scope of the present disclosure. The accompanying method claims present elements of the various steps in a sample sequence, but are not intended to be limited to the specific order or hierarchy presented.

## Claims

1. A method for obtaining molecular diagnostic analysis results, performed by a computer device using a memory, a processor, and one or more programs stored in the memory and configured to be executed by the processor, the method comprising:

   obtaining a dataset representing results of an amplification reaction for a target analyte in a sample;
   calculating a shape similarity for each reference pattern by comparing a target curve generated based on the dataset to each of pre-determined multiple reference patterns; and
   providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

2. The method of claim 1, wherein the amplification reaction is based on real-time amplification.

3. The method of claim 1, wherein the dataset includes a signal value in each of multiple cycles obtained as a result of the amplification reaction or an nth (n is a natural number) derivative result of a curve connecting the signal value in each of the multiple cycles.

4. The method of claim 1, wherein the multiple reference patterns are determined based on at least any one selected from a group including an amplification reference pattern in a case where the target analyte is absent in the sample, an amplification reference pattern in cases where one type of target analyte detectable in a single channel is present at a relatively high concentration or a relatively low concentration in the sample, respectively, an amplification reference pattern in cases where two or more types of target analytes detectable in the single channel are present at same concentration or at different concentrations in the sample, respectively, an aspect of a background signal included in a result of the amplification reaction, an aspect of an abnormal signal included in the result of the amplification reaction, and an aspect of a non-specific signal due to an amplification reaction other than an intended amplification.

5. The method of claim 4, wherein a reference pattern according to the aspect of the abnormal signal includes a reference pattern in at least one of a case where a magnitude of amplitude included in the result of the amplification reaction increases discretely, a case where signal interference is received from another channel, or a case where the magnitude of the amplitude increases linearly.

6. The method of claim 1, wherein the shape similarity for each reference pattern is calculated by computing a cross correlation between the target curve and each of the multiple reference patterns.

7. The method of claim 6, wherein a computation of the cross correlation is performed by at least any one selected from a group including a pre-stored cross correlation scheme, a zero-normalized cross correlation scheme, a normalized cross correlation scheme, and a correlation coefficient scheme.

8. The method of claim 1, wherein the shape similarity for each reference pattern is generated as an image type, and the pre-trained estimation model receives the shape similarity for each reference pattern generated as the image type as an input.

9. The method of claim 8, wherein, in the shape similarity for each reference pattern, a similarity of the target curve with respect to each reference pattern is distinguished by color on the image.

10. The method of claim 8, wherein the shape similarity for each reference pattern of the image type is obtained by measuring the shape similarity at each shift amount for each of the multiple reference patterns, when shifting any one of the target curve and the reference pattern by changing the shift amounts.

11. The method of claim 1, wherein the pre-trained estimation model includes at least any one selected from a group including a convolutional neural network (CNN), a deep neural network (DNN), a recurrent neural network (RNN), a vision transformer (ViT), and a generative adversarial network (GAN).

12. The method of claim 1, wherein a range of the Ct, the quantitative value, the positive/negative determination result for the target analyte in the sample, or the suitability assessment result of the oligonucleotide candidates is partitioned into multiple sections, and each of the multiple sections is mapped to any one of multiple classes, and
when receiving the shape similarity for each reference pattern, the pre-trained estimation model outputs a probability value for each of the multiple classes.

13. The method of claim 1, wherein the pre-trained estimation model is trained using multiple training datasets, and each training dataset includes (a) training input data including the shape similarity for each reference pattern by comparing the target curve generated based on the dataset representing the result of the amplification reaction for the target analyte in the sample to each of the multiple reference patterns, and (b) training ground truth data including label data for the molecular diagnostic analysis results including at least any one selected from a group including the Ct, the quantitative value, the positive/negative determination result for the target analyte in the sample, and the suitability assessment result of the oligonucleotide candidates.

14. A computer program stored in a non-transitory computer-readable storage medium, wherein the computer program, when executed by at least one processor, includes instructions for causing the at least one processor to perform a method for obtaining molecular diagnostic analysis results, the method comprising:

obtaining a dataset representing results of an amplification reaction for a target analyte in a sample;

calculating a shape similarity for each reference pattern by comparing a target curve generated based on the dataset to each of pre-determined multiple reference patterns; and

providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

15. A non-transitory computer-readable storage medium storing a computer program, wherein the computer program, when executed by at least one processor, includes instructions for causing the at least one processor to perform a method for obtaining molecular diagnostic analysis results, the method comprising:

obtaining a dataset representing results of an amplification reaction for a target analyte in a sample;

calculating a shape similarity for each reference pattern by comparing a target curve generated based on the dataset to each of pre-determined multiple reference patterns; and

providing the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/negative determination result for the target analyte in the sample, and a suitability assessment result of oligonucleotide candidates to be used in the amplification reaction.

16. A computer device, comprising:

a memory storing at least one instruction; and
a processor;
wherein the at least one instruction, when executed by the processor, causes the processor to:

obtain a dataset representing results of an amplification reaction for a target analyte in a sample;

calculate a shape similarity for each reference pattern by comparing a target curve generated based on the dataset to each of pre-determined multiple reference patterns; and

provide the shape similarity for each reference pattern to a pre-trained estimation model, to obtain, from the pre-trained estimation model, molecular diagnostic analysis results including at least any one selected from a group including Ct of the target curve, a quantitative value of the target analyte in the sample, a positive/-negative determination result for the target analyte in the sample, and a suitability assessment result of oligonucleotide candidates to be used in the amplification reaction.

17. A method for obtaining a model to estimate molecular diagnostic analysis results, performed by a computer device using a memory, a processor, and one or more programs stored in the memory and configured to be executed by the processor, the method comprising:

obtaining multiple training datasets; each training dataset including (a) training input data including a shape similarity for each reference pattern by comparing a target curve generated based on a dataset representing a result of an amplification reaction for a target analyte in a sample to each of pre-determined multiple reference patterns, and (b) training ground truth data including label data for at least any one selected from a group including Ct, a quantitative value, a positive/negative determination result for the target analyte in the sample, and a suitability assessment result of oligonucleotide candidates; and

obtaining an estimation model trained to estimate at least any one selected from a group including the Ct, the quantitative value, the positive/negative determination result for the target analyte in the sample, and the suitability assessment result of oligonucleotide candidates, when the shape similarity for each reference pattern is provided using the multiple training datasets.

18. A method for obtaining molecular diagnostic analysis results, performed by a computer device using a memory, a processor, and one or more programs stored in the memory and configured to be executed by the processor, the method comprising:

obtaining a dataset representing results of an amplification reaction for a target analyte in a sample;

calculating a shape similarity for each reference pattern by comparing a target curve generated based on the dataset to each of pre-determined multiple reference patterns; and

obtaining molecular diagnostic analysis results for the target analyte in the sample using the shape similarity for each reference pattern.

19. A method of claim 18, whrein the molecular diagnostic analysis results includes at least any one selected from a group including Ct of the target curve, a quantitative value of the target analyte in the sample, a positive/negative determination result for the target analyte in the sample, and a suitability assessment result of oligonucleotide candidates to be used in the amplification reaction.

20. A method for calculating a shape similarity for molecular diagnostic analysis, performed by a computer device using a memory, a processor, and one or more programs stored in the memory and configured to be executed by the processor, the method comprising:

obtaining a dataset representing a result of an amplification reaction; and

calculating a shape similarity for each reference pattern by comparing a target curve generated based on the dataset to each of pre-determined multiple reference patterns.

21. The method of claim 20, wherein the multiple reference patterns are determined based on at least any one selected from a group including an amplification reference pattern in a case where the target analyte is absent in the sample, an amplification reference pattern in cases where one type of target analyte detectable in a single channel is present at a relatively high concentration or a relatively low concentration in the sample, respectively, an amplification reference pattern in cases where two or more types of target analytes detectable in the single channel are present at same concentration or at different concentrations in the sample, respectively, an aspect of a background signal included in a result of the amplification reaction, an aspect of an abnormal signal included in the result of the amplification reaction, and an aspect of a non-specific signal due to an amplification reaction other than an intended amplification.

22. The method of claim 20, wherein the shape similarity for each reference pattern is calculated by computing a cross correlation between the target curve and each of the multiple reference patterns.

23. The method of claim 20, wherein the shape similarity for each reference pattern is generated as an image type, and the pre-trained estimation model receives the shape similarity for each reference pattern generated as the image type as an input.

# FIG.1

# FIG.2

110
MODEL LEARNING UNIT

120
ESTIMATION UNIT

# FIG.3

111～ DATASET . . .

MULTIPLE REFERENCE PATTERNS ～112

SHAPE SIMILARITY FOR EACH REFERENCE PATTERN

113 . . .

## FIG.4A

111a

## FIG.4B

111b

## FIG.4C

111c

## FIG.4D

111d

# FIG.5A

## 112a

# FIG.5B

## 112b

# FIG.5C

## 112c

# FIG.5D

## 112d

# FIG.5E

## 112e

# FIG.6A

# FIG.6B

FIG.7

# FIG.8A

**113b**

REFERENCE PATTERN 1

REFERENCE PATTERN 2

REFERENCE PATTERN 3

REFERENCE PATTERN 4

REFERENCE PATTERN 5

**113**

**113a**

SHIFT AMOUNT FOR CYCLE

# FIG.8B

1

0

EP 4 648 054 A1

## *FIG.9A*

113a

## *FIG.9B*

113b

## *FIG.9C*

113c

## *FIG.9D*

113d

# *FIG.10*

# *FIG.11*

| | TRAINING INPUT DATA | TRAINING GROUND TRUTH DATA |
|---|---|---|
| FIRST TRAINING DATASET | | CLASS 2 |
| SECOND TRAINING DATASET | | CLASS 1 |
| THIRD TRAINING DATASET | | CLASS 3 |
| . . . | . . . | . . . |
| MTH TRAINING DATASET | | CLASS 5 |

# FIG.12

SHAPE SIMILARITY FOR
EACH REFERENCE PATTERN — 113

DEEP LEARNING MODEL — 116

CLASS-SPECIFIC
PROBABILITY VALUE — 117

LABEL DATA FOR
MOLECULAR DIAGNOSTIC
ANALYSIS RESULT — 114

# FIG.13

116a     116b     116c   116d

# FIG.14

START

OBTAIN MULTIPLE TRAINING DATASET — S1101

OBTAIN ESTIMATION MODEL TRAINED TO ESTIMATE
MOLECULAR DIAGNOSTIC ANALYSIS RESULT INCLUDING
AT LEAST ANY ONE SELECTED FROM THE GROUP
INCLUDING CT, QUANTITATIVE VALUE, POSITIVE/NEGATIVE
DETERMINATION RESULT, AND SUITABILITY ASSESSMENT — S1102
RESULT OF OLIGONUCLEOTIDE CANDIDATES, WHEN
SHAPE SIMILARITY FOR EACH REFERENCE PATTERN IS
PROVIDED USING MULTIPLE TRAINING DATASETS

END

# FIG.15

| 121 | 122 |
|---|---|
| DATASET | MULTIPLE REFERENCE PATTERNS |

123
SHAPE SIMILARITY FOR EACH REFERENCE PATTERN

*FIG.16A*

*FIG.16B*

*FIG.16C*

*FIG.16D*

# FIG.17

SHAPE SIMILARITY FOR EACH REFERENCE PATTERN — 123

ESTIMATION MODEL — 124

CLASS-SPECIFIC PROBABILITY VALUE — 125

# FIG.18

TEST SUBJECT → NUCLEIC ACID AMPLIFICATION REACTION → DATASET →

ANALYSIS RESULT OF NUCLEIC ACID AMPLIFICATION REACTION

TEST SUBJECT: HONG GIL DONG

VIRUS SPECIES : SARS-CoV-2

PRESENCE OR ABSENCE OF DETECTION : O

126 — CT ESTIMATION RESULT : 23

QUANTITATION RESULT : XXX (ng)

POSITIVE/NEGATIVE DETERMINATION RESULT : POSITIVE

EP 4 648 054 A1

# *FIG.19*

```
┌─────────────┐
│    START    │
└─────────────┘
       │
       ▼
┌──────────────────────────────────────────────────┐
│  OBTAIN DATASET REPRESENTING RESULT OF           │  ～S1201
│  AMPLIFICATION REACTION                          │
└──────────────────────────────────────────────────┘
       │
       ▼
┌──────────────────────────────────────────────────┐
│  CALCULATE SHAPE SIMILARITY FOR EACH REFERENCE   │
│  PATTERN BY COMPARING TARGET CURVE GENERATED     │  ～S1202
│  BASED ON DATASET TO EACH OF MULTIPLE            │
│  PRE-ESTABLISHED REFERENCE PATTERNS              │
└──────────────────────────────────────────────────┘
       │
       ▼
┌──────────────────────────────────────────────────┐
│  PROVIDE SHAPE SIMILARITY FOR EACH REFERENCE     │
│  PATTERN TO PRE-TRAINED ESTIMATION MODEL AND     │
│  OBTAIN MOLECULAR DIAGNOSTIC ANALYSIS RESULT     │
│  INCLUDING AT LEAST ANY ONE SELECTED FROM THE    │  ～S1203
│  GROUP INCLUDING CT, QUANTITATIVE VALUE,         │
│  POSITIVE/NEGATIVE DETERMINATION RESULT, AND     │
│  SUITABILITY ASSESSMENT RESULT OF OLIGONUCLEOTIDE│
│  CANDIDATES FROM ESTIMATION MODEL                │
└──────────────────────────────────────────────────┘
       │
       ▼
┌─────────────┐
│     END     │
└─────────────┘
```

# *FIG.20*

START

OBTAIN DATASET REPRESENTING RESULT OF
REACTION FOR TARGET ANALYTE IN SAMPLE — S1204

CALCULATE SHAPE SIMILARITY FOR EACH REFERENCE
PATTERN BY COMPARING TARGET CURVE GENERATED
BASED ON DATASET TO EACH OF MULTIPLE
PRE-ESTABLISHED REFERENCE PATTERNS — S1205

OBTAIN MOLECULAR DIAGNOSTIC ANALYSIS RESULT
FOR TARGET ANALYTE IN SAMPLE USING SHAPE
SIMILARITY FOR EACH REFERENCE PATTERN — S1206

END

## INTERNATIONAL SEARCH REPORT

| International application No. |
|---|
| **PCT/KR2023/021950** |

| A. | CLASSIFICATION OF SUBJECT MATTER |
|---|---|

**G16B 40/10**(2019.01)i; **G16B 45/00**(2019.01)i; **G16B 20/00**(2019.01)i; **G06N 3/04**(2006.01)i; **G16B 40/20**(2019.01)i; **G16B 25/20**(2019.01)i

According to International Patent Classification (IPC) or to both national classification and IPC

| B. | FIELDS SEARCHED |
|---|---|

Minimum documentation searched (classification system followed by classification symbols)

G16B 40/10(2019.01); G06F 19/10(2011.01); G06N 20/00(2019.01); G16B 40/00(2019.01); G16B 40/20(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 분자 진단(molecule analysis), 기계 학습 모델(machine learning model), 형상 유사도(shape similarity), 증폭 곡선(amplification curve), 핵산 증폭(nucleic acid amplification)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0000458 A (GENOPLAN KOREA INC.) 04 January 2022 (2022-01-04)<br>See claim 1; and paragraphs [0008]-[0116]. | 1-23 |
| Y | KR 10-2016-0039529 A (SEEGENE, INC.) 11 April 2016 (2016-04-11)<br>See claim 1; and paragraphs [0115] and [0248]-[0264]. | 1-23 |
| A | WO 2022-038279 A1 (IMPERIAL COLLEGE INNOVATIONS LIMITED) 24 February 2022 (2022-02-24)<br>See claim 1; and pages 2-22. | 1-23 |
| A | KR 10-2021-0007735 A (ELECTRONICS AND TELECOMMUNICATIONS RESEARCH INSTITUTE) 20 January 2021 (2021-01-20)<br>See entire document. | 1-23 |
| A | KR 10-2014-0002242 A (SAMSUNG ELECTRONICS CO., LTD.) 08 January 2014 (2014-01-08)<br>See entire document. | 1-23 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 April 2024** | **16 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/021950**

| Patent document cited in search report | | | Publication date (day/month/year) | Patent family member(s) | | | Publication date (day/month/year) |
|---|---|---|---|---|---|---|---|
| KR | 10-2022-0000458 | A | 04 January 2022 | KR | 10-2470870 | B1 | 25 November 2022 |
| KR | 10-2016-0039529 | A | 11 April 2016 | EP | 3201359 | A1 | 09 August 2017 |
| | | | | JP | 2018-502552 | A | 01 February 2018 |
| | | | | KR | 10-2017-0051539 | A | 11 May 2017 |
| | | | | KR | 10-2336732 | B1 | 08 December 2021 |
| | | | | US | 2017-0226563 | A1 | 10 August 2017 |
| | | | | US | 2021-0238652 | A1 | 05 August 2021 |
| | | | | WO | 2016-052991 | A1 | 07 April 2016 |
| WO | 2022-038279 | A1 | 24 February 2022 | EP | 4200861 | A1 | 28 June 2023 |
| | | | | US | 2023-0326553 | A1 | 12 October 2023 |
| KR | 10-2021-0007735 | A | 20 January 2021 | KR | 10-2461731 | B1 | 01 November 2022 |
| | | | | US | 11880773 | B2 | 23 January 2024 |
| | | | | US | 2021-0012213 | A1 | 14 January 2021 |
| KR | 10-2014-0002242 | A | 08 January 2014 | US | 2014-0005948 | A1 | 02 January 2014 |

Form PCT/ISA/210 (patent family annex) (July 2022)

## REFERENCES CITED IN THE DESCRIPTION

**Patent documents cited in the description**

- US 4683195 A, Mullis
- US 4683202 A
- US 4800159 A

- KR 102050601
- US 8560247 B

**Non-patent literature cited in the description**

- **SAIKI et al.** *Science*, 1985, vol. 230, 1350-1354

- **SAMBROOK, J. et al.** Molecular Cloning. A Laboratory Manual. Cold Spring Harbor Press, 2001